# FASCICULE DE BREVET EUROPEEN

(11) **EP 3 077 527 B1**
(45) Date de publication et mention de la délivrance du brevet: **29.05.2019**
(21) Numéro de dépôt: 14806674.9
(22) Date de dépôt: 05.12.2014
(51) Int. Cl.: C12Q 1/26, C12N 9/02

(54) **MICROSOMES IRREVERSIBLEMENT INHIBES POUR DES CYP450 LEURS UTILISATIONS DANS LE PHENOTYPAGE ENZYMATIQUE DES VOIES METABOLIQUES**
IRREVERSIBEL GEHEMMTE MIKROSOMEN FÜR CYP450, IHRE EINSATZMÖGLICHKEITEN BEI DER ENZYMATISCHEN PHÄNOTYPISIERUNG DER STOFFWECHSELWEGE
IRREVERSIBLY INHIBITED MICROSOMES FOR CYP450 AND USE OF SAME IN ENZYMATIC PHENOTYPING OF THE METABOLIC PATHWAYS

(30) Priorité: 05.12.2013 EP 13306674; 05.12.2013 US 201361912186 P
(43) Date de publication de la demande: 12.10.2016
(73) Titulaire: Les Laboratoires Servier, 92284 Suresnes Cedex (FR)
(72) Inventeur: CARADEC, Fabrice, F-45450 Donnery (FR); PARMENTIER, Yannick, F-45560 Saint-Denis-en-Val (FR); POTHIER, Corinne, F-45800 Saint-Jean-de-Braye (FR)
(86) Numéro de dépôt international: PCT/EP2014/076748
(87) Numéro de publication internationale: WO 2015/082694

(56) Documents cités:
- WO-A1-2006/114275
- JI-YOON LEE ET AL: "Assessment of drugdrug interactions caused by metabolism-dependent cytochrome P450 inhibition", CHEMICO-BIOLOGICAL INTERACTIONS, ELSEVIER SCIENCE IRLAND, IR, vol. 198, no. 1, 21 mai 2012 (2012-05-21), pages 49-56, XP028500887, ISSN: 0009-2797, DOI: 10.1016/J.CBI.2012.05.007 [extrait le 2012-05-28]
- D. M. STRESSER ET AL: "HIGHLY SELECTIVE INHIBITION OF HUMAN CYP3A IN VITRO BY AZAMULIN AND EVIDENCE THAT INHIBITION IS IRREVERSIBLE", DRUG METABOLISM AND DISPOSITION, vol. 32, no. 1, 1 janvier 2004 (2004-01-01), pages 105-112, XP055118730, ISSN: 0090-9556, DOI: 10.1124/dmd.32.1.105
- VICTORIA A. EAGLING ET AL: "Differential selectivity of cytochrome P450 inhibitors against probe substrates in human and rat liver microsomes", BRITISH JOURNAL OF CLINICAL PHARMACOLOGY, vol. 45, no. 2, 1 février 1998 (1998-02-01), pages 107-114, XP055119156, ISSN: 0306-5251, DOI: 10.1046/j.1365-2125.1998.00679.x
- TIMOTHY W. HARPER ET AL: "Reaction Phenotyping: Current Industry Efforts to Identify Enzymes Responsible for Metabolizing Drug Candidates", THE AAPS JOURNAL, vol. 10, no. 1, 1 mars 2008 (2008-03-01), pages 200-207, XP055118740, DOI: 10.1208/s12248-008-9019-6
- SIMON GATES ET AL: "Cytochrome P450 isoform selectivity in human hepatic theobromine metabolism", BRITISH JOURNAL OF CLINICAL PHARMACOLOGY, vol. 47, no. 3, 1 mars 1999 (1999-03-01), pages 299-305, XP055119074, ISSN: 0306-5251, DOI: 10.1046/j.1365-2125.1999.00890.x
- X. LI ET AL: "Discovery of a Highly Selective CYP3A4 Inhibitor Suitable for Reaction Phenotyping Studies and Differentiation of CYP3A4 and CYP3A5", DRUG METABOLISM AND DISPOSITION, vol. 40, no. 9, 13 juin 2012 (2012-06-13), pages 1803-1809, XP055118733, DOI: 10.1124/dmd.112.046144
- FONTANA E ET AL: "Cytochrome p450 enzymes mechanism based inhibitors: common sub-structures and reactivity", CURRENT DRUG METABOLISM, BENTHAM SCIENCE PUBLISHERS, US, vol. 6, no. 5, 1 octobre 2005 (2005-10-01) , pages 413-454, XP009177398, ISSN: 1389-2002
- CLARKE S E: "In vitro assessment of human cytochrome P450", XENOBIOTICA, TAYLOR AND FRANCIS, LONDON, GB, vol. 28, no. 12, 1 décembre 1998 (1998-12-01), pages 1167-1202, XP009177416, ISSN: 0049-8254
- ROBIN E. PEARCE ET AL: "Effects of Freezing, Thawing, and Storing Human Liver Microsomes on Cytochrome P450 Activity", ARCHIVES OF BIOCHEMISTRY AND BIOPHYSICS, vol. 331, no. 2, 15 juillet 1996 (1996-07-15), pages 145-169, XP055118743, ISSN: 0003-9861, DOI: 10.1006/abbi.1996.0294
- D. A. FAIRMAN ET AL: "Progress Curve Analysis of CYP1A2 Inhibition: A More Informative Approach to the Assessment of Mechanism-Based Inactivation?", DRUG METABOLISM AND DISPOSITION, vol. 35, no. 12, 4 septembre 2007 (2007-09-04), pages 2159-2165, XP055137998, ISSN: 0090-9556, DOI: 10.1124/dmd.107.017236
- EVAN P GALLAGHER ET AL: "The Kinetics of Aflatoxin B 1 Oxidation by Human cDNA-Expressed and Human Liver Microsomal Cytochromes P450 1A2 and 3A4 by DNA binding studies. AFB 1 -DNA binding in control HLMs The Kinetics of Aflatoxin B 1 Oxidation by Human cDNA-(reflecting the contribution of CYP1A2 and CYP3A4) and furafyl- Exp", TOXICOL. APPL. PHARMACOL, vol. 141, 1 janvier 1996 (1996-01-01), pages 595-606, XP055138000,
- BROOKE M VANDENBRINK ET AL: "The role of metabolites in predicting drug-drug interactions: focus on irreversible cytochrome P450 inhibition", CURRENT OPINION IN DRUG DISCOVERY & DEVELOPMENT, vol. 13, no. 1, 1 janvier 2010 (2010-01-01), pages 66-77, XP055138168, England

## Description

### Domaine technique de l'invention

L'invention s'inscrit dans le cadre de la recherche et de la mise au point de produits et méthodes pour l'évaluation des interactions médicamenteuses des nouveaux médicaments. La présente invention concerne une méthode de préparation de microsomes isolés irréversiblement inhibés pour un cytochrome P450 humain spécifique (CYP450) qui seront utilisés pour quantifier la contribution de cette enzyme dans le métabolisme de principes actifs.

L'efficacité et la toxicité d'un médicament peuvent être modifiées par l'administration d'un autre composé : médicament, polluant environnemental, aliment. Il s'agit d'interactions médicamenteuses (DDI : Drug Drug Interactions). Il existe différents types de mécanismes d'interactions, la plus importante étant l'interaction métabolique appartenant au groupe des interactions pharmacocinétiques.

Par interaction médicamenteuse métabolique on entend entre autre le fait qu'un médicament A peut modifier le métabolisme d'un médicament B co-administré soit en accélérant le métabolisme de B (activation ou induction) soit en le réduisant (inhibition ou répression). De cette interaction médicamenteuse métabolique va découler dans le programme de développement d'un nouveau médicament d'une part, l'identification des enzymes impliquées dans le métabolisme du principe actif et d'autre part, le potentiel inhibiteur ou inducteur dudit principe actif.

Le foie est le principal site du métabolisme des médicaments. L'hépatocyte contient des enzymes essentielles du métabolisme parmi lesquelles les cytochromes P450 (CYP450). Les cytochromes P450 constituent donc la cible majeure dans la prédiction des interactions médicamenteuses.

Afin de prédire le risque d'interactions médicamenteuses, il faut identifier et déterminer la contribution de chaque enzyme dans le métabolisme du principe actif : c'est le phénotypage des réactions enzymatiques.

Pour estimer la contribution de chaque enzyme dans le métabolisme du principe actif, différentes méthodes de phénotypages des voies métaboliques peuvent être mises en oeuvre.

L'utilisation d'une banque de microsomes de foies humains caractérisés peut aider à l'identification des enzymes impliquées dans le métabolisme d'un principe actif. Cette méthode nécessite une caractérisation préalable de l'activité enzymatique des principaux CYP450 d'au moins quinze lots individuels de microsomes issus de foies humains. Chacun des quinze lots de microsomes est incubé avec le principe actif afin de déterminer une corrélation entre sa vitesse de métabolisme et l'activité de chaque cytochrome P450 de ces mêmes microsomes. Cependant l'utilisation des banques microsomales ne permet pas une détermination quantitative des enzymes impliquées, la corrélation étant difficile à réaliser.

Les enzymes recombinantes sont également utilisées pour estimer la contribution relative de chaque cytochrome P450 dans le métabolisme du principe actif. Le niveau d'expression du cytochrome P450 dans le système recombinant étant différent, souvent beaucoup plus élevé, que dans les microsomes de foie humain natif, il est nécessaire d'intégrer un facteur correctif pour extrapoler la contribution de chaque CYP450 dans les microsomes recombinants par rapport aux microsomes humains (facteur de correction = Relative Activity Factor). Ce facteur de correction doit être calculé par mesure expérimentale de l'activité enzymatique de chaque CYP450 testé d'une part, sur les microsomes humains natifs et d'autre part, sur les microsomes recombinants. Cette approche permet d'évaluer indirectement de manière sélective et semi-quantitative la contribution relative de chaque enzyme dans le métabolisme du principe actif. Cependant de par leurs caractéristiques intrinsèques ces enzymes recombinantes diffèrent des enzymes retrouvées dans les microsomes de foie (séquence protéique tronquée, environnement membranaire distinct, couplage différent entre les cytochromes b5 et P450, absence de compétition entre les différents CYP450).

Les inhibiteurs biologiques, tels que les anticorps monoclonaux, dirigés contre une enzyme sont utilisés, après co-incubation du principe actif avec les microsomes de foie humain (versus un témoin sans anticorps) pour une estimation quantitative du métabolisme du principe actif. Cependant un nombre non négligeable d'anticorps utilisés montrent un manque de spécificité et de puissance d'inhibition. Enfin cette technique est lourde à mettre en place pour le phénotypage de voies métaboliques.

Les inhibiteurs chimiques des CYP450 permettent de déterminer la contribution de chaque cytochrome P450 directement par le pourcentage d'inhibition du métabolisme du principe actif si l'inhibition est totale et spécifique de l'enzyme étudiée. Les inhibiteurs peuvent se fixer de manière réversible ou non réversible à l'enzyme. Ils sont utilisés en co-incubation ou pré-incubation avec le principe actif et les microsomes de foie humain (versus une condition témoin sans inhibiteur), modèle représentatif de l*'in vivo* pour les voies oxydatives de métabolisme des principes actifs.

Le niveau d'inhibition liée aux inhibiteurs réversibles de type compétitifs (les plus fréquents) est dépendant des conditions d'incubations telles que le temps d'incubation et de la concentration en substrat. De plus, de nombreux inhibiteurs de CYP450 utilisés communément pour les études de phénotypage ne sont pas spécifiques d'un seul CYP450 (ex : kétoconazole, quercetine...). En conséquence, les inhibiteurs réversibles ne conviennent pas au phénotypage des cytochromes P450.

Afin de pallier aux inconvénients de ces méthodes, les inhibiteurs non réversibles ou irréversibles sont utilisés en vue d'obtenir une méthode quantitative robuste et représentative des conditions *in vivo.* L'inhibition est dite irréversible dans la mesure où l'enzyme ne retrouve jamais son activité, on parle d'inhibition « suicide ». Les inhibiteurs non réversibles sont oxydés par les cytochromes P450 en métabolites intermédiaires se liant irréversiblement aux enzymes. Ce processus est qualifié de MBI pour Mechanism-Based Inhibition car la molécule de départ n'est pas inhibitrice mais nécessite au moins un cycle catalytique de l'enzyme avant d'être activée en métabolite réactif se liant de manière covalente à ladite enzyme. Une inhibition MBI se caractérise par une inhibition irréversible du cytochrome P450 étudié et ne dépend pas de la concentration en substrat. Ces inhibiteurs suivent une cinétique de premier ordre avec les constantes suivantes : k_{inact} correspond à la vitesse maximale d'inactivation de l'enzyme et K_{I} correspond à la concentration en inhibiteur à la moitié de la vitesse maximale d'inactivation.

Plusieurs conditions doivent être réunies pour obtenir une inhibition totale d'un CYP450 en utilisant un inhibiteur irréversible :
1) il est nécessaire d'utiliser une concentration en inhibiteur non réversible suffisante (dépendant de son K_{I}) ;
2) dépendant de son k_{inact}, le temps de pré-incubation de l'inhibiteur avec les microsomes de foies doit être suffisant pour entraîner suffisamment de cycles catalytiques d'inactivation ;
3) le temps de pré-incubation ne doit pas entraîner de déplétion en inhibiteur suicide tel que sa concentration deviendrait trop faible pour inhiber totalement le P450.

Si l'inhibition du CYP450 est totale et spécifique (aucun autre P450 inhibé) il est donc possible de déterminer de manière quantitative l'implication de chaque cytochrome P450 dans le métabolisme d'un principe actif. Le schéma expérimental utilisé est alors le suivant :

| Sequence | | Microsomes de foie | Conditions de préincubation | Microsomes irréversiblement inhibés | Conditions d'incubations | f(t) =[PA] |
|---|---|---|---|---|---|---|
| | | | 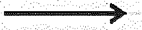 | | 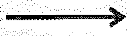 | |
| | | | [NADPH] 't' Θ' | | [NADPH] 't' Θ' | |
| Essai PA | Essai | + MBI | | + PA | | |
| | Témoin | - MBI | | + PA | | |
| Contrôle + Substrat spécifique | Essai | + MBI | | + Substrat | | |
| | Témoin | - MBI | | + Substrat | | |

En dépit des avantages cités ci-dessus, l'utilisation des MBI selon ce schéma expérimental présente un certains nombres de contraintes qui limitent son intérêt :
- d'une part, les conditions d'incubations du principe actif étudié sont dépendantes des conditions optimales de pré-incubation de l'inhibiteur non réversible (concentrations en protéines microsomales, pourcentage de solvant organique). En effet, afin de mesurer précisément le pourcentage d'inhibition du métabolisme du principe actif, ce dernier doit être incubé dans les conditions dites initiales (linéarité de la vitesse du métabolisme en fonction du temps et des concentrations en protéines, pourcentage de solvant ne peut excéder un certain niveau). Or, pour obtenir une inhibition maximale et spécifique dans ce schéma expérimental linéaire, l'inhibiteur doit lui-même être préalablement incubé dans des conditions *in vitro* de concentrations en protéines ou de solvant compatibles avec l'incubation du principe actif.
- d'autre part, le temps de pré-incubation altère l'activité enzymatique des CYP450 de microsomes qui présentent une demi-vie *in vitro* d'environ 70 à 90 minutes. On observe par exemple une perte d'activité enzymatique CYP2D6 jusqu'à 26% lors d'une pré-incubation de 30 minutes, jusqu'à 35% pour une pré-incubation de 40 minutes et 46% pour une pré-incubation de 60 minutes. En conséquence, la durée de la séquence continue de pré-incubation du MBI et d'incubation du candidat médicament imposée par le schéma expérimental est donc incompatible avec la demi-vie *in vitro* des CYP450 de microsomes.
- enfin, il est fréquent que les inhibiteurs irréversibles pour un CYP450 présentent également une inhibition réversible pour un ou plusieurs CYP450 (par exemple l'inhibiteur MBI du CYP1A2 est également inhibiteur compétitif du CYP2C19 et l'inhibiteur MBI du CYP2B6 est aussi inhibiteur compétitif des CYP2A6 et 3A4). Or le schéma expérimental linéaire ci-dessus ne permet pas d'éliminer de manière satisfaisante pour une étude de phénotypage *in vitro,* la part libre restante d'inhibiteur pouvant agir de manière compétitive sur d'autres CYP450. Une dilution du pré-incubat peut diminuer la concentration en inhibiteur libre, d'où moins d'inhibition réversible non spécifique, cependant elle va de facto diminuer la concentration en protéines microsomales de telle façon que le métabolisme du principe actif lors de l'incubation sera trop faible pour être détecté.

En conséquence, les difficultés pour rendre compatibles les conditions d'incubations de l'inhibiteur irréversible d'une part et du principe actif d'autre part, sont telles qu'il est rarement possible d'utiliser ce schéma expérimental linéaire.

De plus, lors de ce schéma expérimental linéaire, il est nécessaire d'ajouter deux séries d'incubations additionnelles (avec et sans inhibiteur irréversible) avec un substrat spécifique du CYP450 testé comme contrôle positif validant l'effet de l'inhibiteur. Ceci impose donc de quantifier non seulement le principe actif mais également l'ensemble des substrats spécifiques, et cela pour autant de CYP450 à tester, ce qui alourdi considérablement l'expérimentation.

Compte-tenu des inconvénients respectifs des méthodes de phénotypage des réactions enzymatiques impliquées dans le métabolisme d'un principe actif, une étude *in vitro* dudit phénotypage des réactions enzymatiques exige de surmonter les désavantages des différentes méthodes précédemment décrites.

### Description détaillée de l'invention

La présente invention a donc pour but de proposer une stratégie alternative permettant de surmonter les problèmes inhérents à la mise en oeuvre d'une étude de phénotypage des réactions enzymatiques impliquées dans le métabolisme d'un principe actif par l'utilisation de microsomes isolés irréversiblement inhibés.

Cette invention a pour objet une méthode de préparation de microsomes isolés et conservés qui comprennent un cytochrome P450 (CYP450) irréversiblement inhibé pour leur utilisation extemporanée et comprenant les étapes suivantes :
- l'inhibition irréversible du cytochrome P450 ;
- la concentration des protéines microsomales ; et
- la conservation des microsomes isolés.

Par inhibiteur non réversible, inhibiteur irréversible, inhibiteur MBI, inhibition irréversible ou inhibition suicide on entend un inhibiteur capable de se fixer de manière covalente à une enzyme ; l'enzyme ainsi inhibée ne peut retrouver son activité fonctionnelle initiale. De manière plus spécifique un inhibiteur MBI (Mechanism Based Inhibition) ne forme pas directement une liaison irréversible avec l'enzyme mais un de ses métabolites intermédiaires réactifs se lie de manière covalente à l'enzyme.

La concentration selon l'invention a pour but, d'une part de filtrer les protéines microsomales des autres produits présents dans la préparation notamment l'inhibiteur irréversible en excès restant libre ainsi que les solvants, d'autre part de concentrer les microsomes dilués par l'étape de pré-incubation.

La concentration des protéines microsomales selon l'invention peut être obtenue par filtration puis centrifugation. La fonction de filtration dans l'étape de concentration est obtenue à partir d'une membrane avec un seuil de coupure compris entre 10 000 Daltons et 40 000 Daltons, de préférence 30 000 Daltons. La fonction de concentration dans l'étape de concentration est réalisée par un système permettant la concentration, par exemple un système Centricon® soumis à une centrifugation de 3000 g à 4000 g pendant 60 à 90 minutes, de préférence pendant 80 minutes. A la fin de cette étape, le Centricon® est retourné et centrifugé de 800 à 1000 g pendant 2 à 10 minutes, de préférence pendant 5 minutes.

Afin d'améliorer la séparation et la concentration des protéines microsomales, une étape supplémentaire d'ultracentrifugation dans les conditions suivantes de 80 000 g à 150 000 g pendant environ 60 minutes peut être effectuée.

La concentration des protéines microsomales peut également être améliorée en répétant au moins deux fois la séquence : filtration puis concentration, décrite ci-dessus.

L'étape de concentration des protéines microsomales selon l'invention peut également être obtenue par ultracentrifugations successives. Au moins deux ultracentrifugations successives sont nécessaires dans les conditions suivantes de 80 000g à 150 000g pendant 60 à 90 minutes.

L'invention concerne une méthode de préparation de microsomes isolés irréversiblement inhibés dans laquelle une ou plusieurs étapes de lavage sont ajoutées. Cette méthode de préparation des microsomes isolés comprend des étapes de lavage placées avant et/ou après l'étape de concentration des protéines microsomales. Par exemple, la méthode selon l'invention comprend les étapes suivantes :
- l'inhibition irréversible d'un cytochrome P450 ;
- le lavage ;
- la concentration des protéines microsomales ;
- le lavage des protéines microsomales.

La méthode selon l'invention peut également comprendre les étapes suivantes :
- l'inhibition irréversible d'un cytochrome P450 ;
- la concentration des protéines microsomales ;
- le lavage des protéines microsomales ;
ou les étapes suivantes :
- l'inhibition irréversible d'un cytochrome P450 ;
- le lavage ;
- la concentration des protéines microsomales.

Par lavage on entend une étape rinçage et élimination des fractions non microsomales. L'étape de lavage peut consister également à reprendre le concentrat de microsomes dans du tampon Tris HCl à pH 7.4.

A l'issue de la méthode de préparation des microsomes isolés irréversiblement inhibés selon l'invention, les microsomes sont à une concentration de 10mg/ml à 30mg/ml, de manière plus spécifique de 17mg/ml à 25mg/ml et de préférence de 20mg/ml. Les protéines microsomales ont été concentrées par un facteur 5 à 15.

La méthode de préparation de microsomes isolés inactivés selon la présente invention comprend une étape finale de conservation des microsomes. L'étape de conservation des microsomes consiste de préférence en une étape de cryoconservation consistant à refroidir à très basse température (environ -196°C), en présence ou non de solutions cryoprotectrices, les microsomes isolés et purifiés afin de suspendre toute activité biologique. Cette étape de conservation a pour but de faciliter l'utilisation *ex temporané* des microsomes inactivés et isolés selon l'invention. Cette utilisation des microsomes isolés peut avoir lieu de quelques jours à plusieurs mois après leur préparation. L'étape de conservation n'altère aucunement les propriétés structurelles et fonctionnelles des microsomes.

De manière préférée, l'étape de conservation est une étape de congélation. La congélation des microsomes isolés et irréversiblement inhibés est réalisée à -80°C.

Les microsomes utilisés au cours de la méthode de préparation sont des microsomes de foie humains, de rat, de souris, de porc ou de singe. De préférence, les microsomes sont issus de foies humains contenant l'ensemble des enzymes P450. Afin de tenir compte de la variabilité interindividuelle, les microsomes proviennent de plusieurs donneurs pour constituer un pool de microsomes.

Les cytochromes P450 sont une vaste famille d'isoenzymes constituée de 18 sous familles (CYP1, CYP2, CYP3, CYP4, CYP5, CYP7, CYP8, CYP11, CYP17, CYP19, CYP20, CYP21, CYP24, CYP26, CYP27, CYP39, CYP46, CYP51). La présente invention porte sur une méthode de préparation de microsomes isolés dont le cytochrome P450 irréversiblement inhibé est sélectionné parmi les familles de cytochrome CYP1, CYP2, CYP3 et CYP4.

Le cytochrome P450 irréversiblement inhibé est sélectionné parmi le CYP1A1, CYP1A2, CYP2A6, CYP2B6, CYP2C8, CYP2C9, CYP2C19, CYP2D6, CYP2E1, CYP2J2, CYP3A4, CYP3A5 et CYP4F2.

De préférence, le cytochrome P450 irréversiblement inhibé est sélectionné parmi le CYP1A2, CYP2A6, CYP2B6, CYP2C8, CYP2C9, CYP2C19, CYP2D6, CYP2E1 et CYP3A4.

Parmi les inhibiteurs non réversibles utilisés dans la méthode de préparation de microsomes selon l'invention on peut citer à titre indicatif et non limitatif :

| **CYP** | **Inhibiteurs MBI** | **Paramètres *in vitro (Littérature)*** | |
|---|---|---|---|
| | | ***K_{I} (µM)*** | ***k inact (min-1)*** |
| 1A2 | Furafylline | 3-6 | 0.27-0.87 |
| 2A6 | 8 MethOxyPsoralene | 0.3-1.9 | 0.5-2.1 |
| | Menthofuran | 0.84-2.5 | 0.22-0.25 |
| | Sporalen | 0.6 | 0.3 |
| 2B6 | Thiotepa | 4.8-50 | 0.1-0.2 |
| | Phencyclidine | 10 | 0.01 |
| 2C8 | Gemfibrozil gluc. | 20 | 0.21 |
| 2C9 | Ac. Tienilique | 0.13-20 | 0.05-2 |
| | Suprofen | 3.7-26 | 0.07-0.09 |
| 2C19 | Fluoxetine | 0.2-54 | 0.06-0.1 |
| | Clopidogrel | 0.5-14.3 | 0.056-0.35 |
| | Ticlopidine | 1.65-87 | 0.19-0.032 |
| | Protopine | 7.1 | 0.24 |
| 2D6 | Paroxetine | 0.8-9.32 | 0.17 |
| | MDMA | 23 | 0.18 |
| | EMTPP | 5.5 | 0.09 |
| 2E1 | DiethylDithioCarbamate | 12.2 | 0.02 |
| | Phenethyl isothiocyanate | 9.98 | 0.33 |
| 3A4 | TroleAndOmycine | 2.4 | 0.032 |
| | Diltiazem | 8.7 | 0.005 |
| | Azamulin | 0.2 | 0.7 |

La divulgation porte également sur les microsomes isolés en tant que tels comprenant un cytochrome P450 irréversiblement inhibé.

Les microsomes isolés selon la divulgation comprennent un cytochrome irréversiblement inhibé sélectionné parmi les familles de cytochromes CYP1, CYP2, CYP3 et CYP4.

La divulgation concerne des microsomes isolés dont un cytochrome sélectionné dans la liste suivante est irréversiblement inhibé: CYP1A1, CYP1A2, CYP2A6, CYP2B6, CYP2C8, CYP2C9, CYP2C19, CYP2D6, CYP2E1, CYP2J2, CYP3A4, CYP3A5 et CYP4F2.

Plus particulièrement, la divulgation concerne des microsomes isolés dont un cytochrome sélectionné dans la liste suivante est irréversiblement inhibé: CYP1A2, CYP2A6, CYP2B6, CYP2C8, CYP2C9, CYP2C19, CYP2D6, CYP2E1 et CYP3A4.

De préférence, les microsomes isolés irréversiblement inhibés selon la divulgation sont cryoconservés ou cryoprotégés.

La présente divulgation concerne également les microsomes isolés irréversiblement inhibés sous une forme congelée pour une utilisation *ex temporané* après décongélation.

Les microsomes isolés sont obtenus selon les méthodes de préparation décrites dans la présente demande de brevet.

La divulgation concerne l'utilisation des microsomes isolés irréversiblement inhibés dans le phénotypage des réactions enzymatiques impliquées dans le métabolisme d'un principe actif à évaluer.

La divulgation porte également sur une méthode de phénotypage des réactions enzymatiques impliquées dans le métabolisme d'un principe actif. Cette méthode de phénotypage comprend les étapes suivantes :
- l'incubation de microsomes isolés et irréversiblement inhibés selon l'invention avec un principe actif à évaluer ;
- la mesure de la contribution du cytochrome P450 irréversiblement inhibé impliqué dans le métabolisme du principe actif.

L'incubation est réalisée en présence du principe actif avec d'une part les microsomes de foie isolés, irréversiblement inhibés selon l'invention et d'autre part les microsomes de foie isolés, non inhibés et préparés selon l'invention (témoin). L'incubation est suivie soit à un temps final d'incubation soit sous-forme d'une cinétique, i.e. plusieurs temps d'incubation.

A chaque temps d'incubation, le principe actif et/ou ses métabolites sont quantifiés.

L'activité métabolique (A) du principe actif est mesurée soit via la clairance intrinsèque métabolique (dans le cas d'une cinétique) soit via la vitesse de disparition de principe actif inchangé ou d'apparition de métabolites (si un seul temps final d'incubation a été prévu) dans les deux conditions, inhibée (activité Ai) et non inhibée (témoin avec l'activité A).

Le pourcentage d'inhibition de l'activité métabolique du principe actif se calcule de la manière suivante : pourcentage d'inhibition = (A-Ai)/A. Le pourcentage d'inhibition calculé correspond directement à la contribution du cytochrome P450 irréversiblement inhibé impliqué dans le métabolisme du principe actif.

Enfin la divulgation concerne un kit de phénotypage comprenant :
- des microsomes isolés et irréversiblement inhibés selon l'invention ;
   - des microsomes témoins.

On entend par microsomes témoins, des microsomes ayant subi la méthode de préparation selon l'invention en l'absence de l'inhibiteur irréversible.

De façon préférée, le kit de phénotypage comprend d'une part des microsomes isolés irréversiblement inhibés et cryoconservés et d'autre part des microsomes témoins pouvant également être cryoconservés.

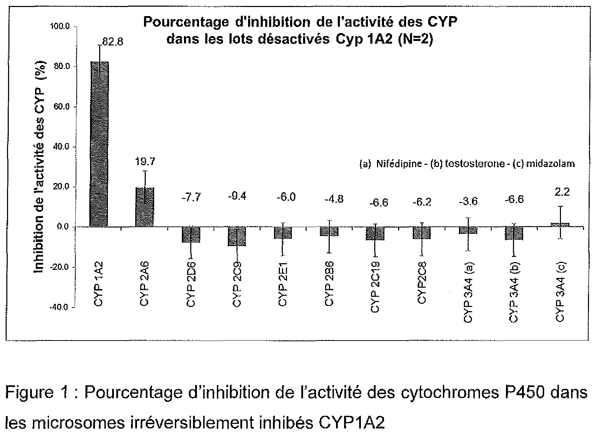

Figure 1 : Pourcentage d'inhibition de l'activité des cytochromes P450 dans les microsomes irréversiblement inhibés CYP1A2.
   Les activités spécifiques étudiées pour chaque CYP450 correspondent aux activités de phénacétine-O-déacéthylase (CYP1A2, incubation de phénacétine à 4.5 µM), coumarin-7-hydroxylase (CYP2A6, incubation de coumarine à 2 µM), bupropion-hydroxylase (2B6, incubation de bupropion à 50 µM), paclitaxel-6α-hydroxylase (2C8, incubation de paclitaxel à 4 µM), diclofenac-4'-hydroxylase (2C9, incubation de diclofenac à 4 µM), omeprazole-5-hydroxylase (2C19, incubation d'oméprazole à 5 µM), dextromethorphan-O-déméthylase (2D6, incubation de dextromethorphan à 5 µM), chlorzoxazone-6-hydroxylase (2E1, incubation de chlorzoxazone à 40 µM) et testostérone-6β-hydroxylase, midazolam-1'-hydroxylase et nifédipine-réductase (3A4, incubation de testostérone à 30 µM, midazolam à 0.5 µM, nifédipine à 10 µM). Le pourcentage d'inhibition est obtenu par comparaison des activités P450 sur microsomes inhibés irréversiblement par la furafylline et sur les microsomes témoins.
Figure 2 : Impact de la furafylline à 5 µM et 10 µM sur les microsomes de foies humains et sur les microsomes recombinants pour le CYP1A2.
Figure 3 : Pourcentage d'inhibition de l'activité des cytochromes P450 dans les microsomes irréversiblement inhibés CYP3A4.
   Les activités spécifiques étudiées pour chaque CYP450 correspondent à celles décrites à la Figure 1. Le pourcentage d'inhibition est obtenu par comparaison des activités P450 sur microsomes inhibés irréversiblement par l'azamuline et sur les microsomes témoins.
Figure 4 : Métabolisme de la nifedipine et du midazolam par le CYP3A4 et le CYP3A5 (microsomes recombinants).
Figure 5 : Pourcentage d'inhibition de l'activité midazolam au Km et au Vmax dans les microsomes irréversiblement inhibés CYP3A4.
Figure 6 : Pourcentage d'inhibition de l'activité des cytochromes P450 dans les microsomes irréversiblement inhibé CYP2C8.
   Les activités spécifiques étudiées pour chaque CYP450 correspondent à celles décrites à la Figure 1 auxquelles s'ajoute l'activité de l'amodiaquine hydroxylase (2C8, incubation d'amodiaquine à 0.5 µM). Le pourcentage d'inhibition est obtenu par comparaison des activités P450 sur microsomes inhibés irréversiblement par le gemfibrozil glucuronide et sur les microsomes témoins.

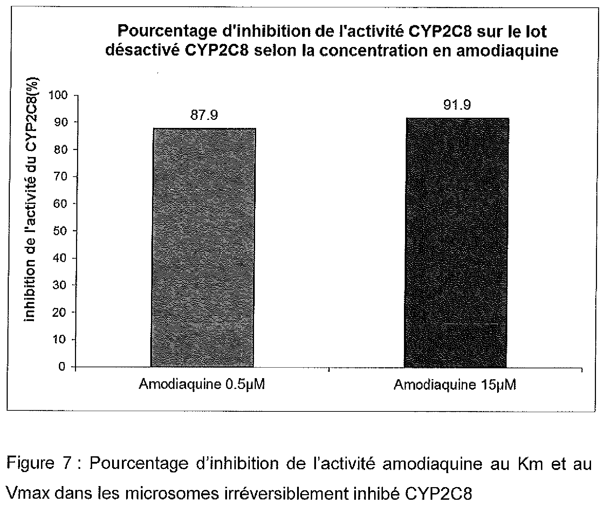

Figure 7 : Pourcentage d'inhibition de l'activité amodiaquine incubé aux concentrations correspondant au Km et au Vmax de la réaction 2C8 dépendante de l'amodiaquine hydroxylation, dans les microsomes irréversiblement inhibé CYP2C8.

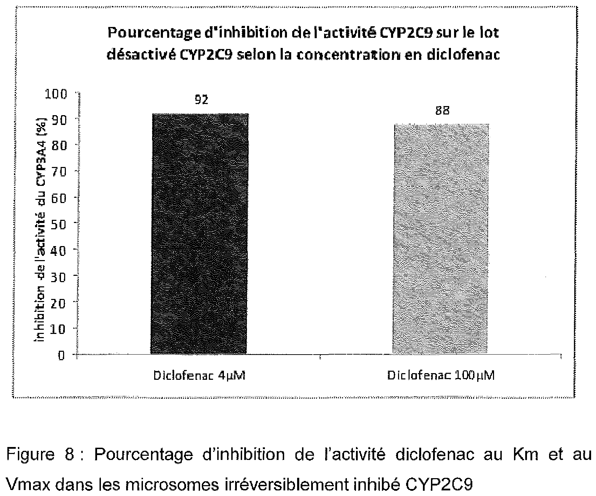

Figure 8 : Pourcentage d'inhibition de l'activité diclofenac incubé aux concentrations correspondant au Km et au Vmax de la réaction 2C9 dépendante de la diclofenac-4'-hydroxylation dans les microsomes irréversiblement inhibé CYP2C9.

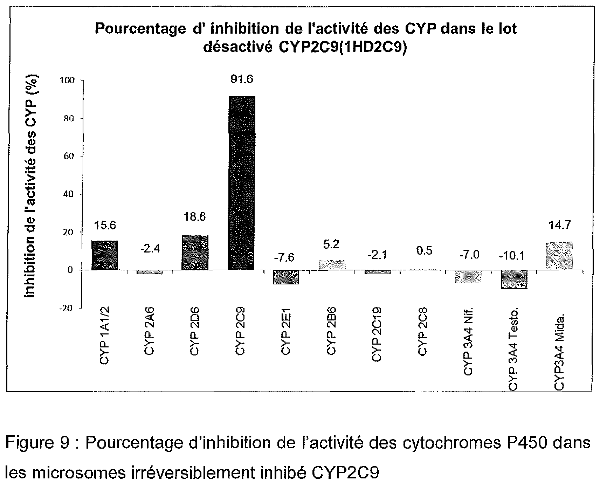

Figure 9 : Pourcentage d'inhibition de l'activité des cytochromes P450 dans les microsomes irréversiblement inhibé CYP2C9.
   Les activités spécifiques étudiées pour chaque CYP450 correspondent à celles décrites à la Figure 1. Le pourcentage d'inhibition est obtenu par comparaison des activités P450 sur microsomes inhibés irréversiblement par l'acide tienilique et sur les microsomes témoins.

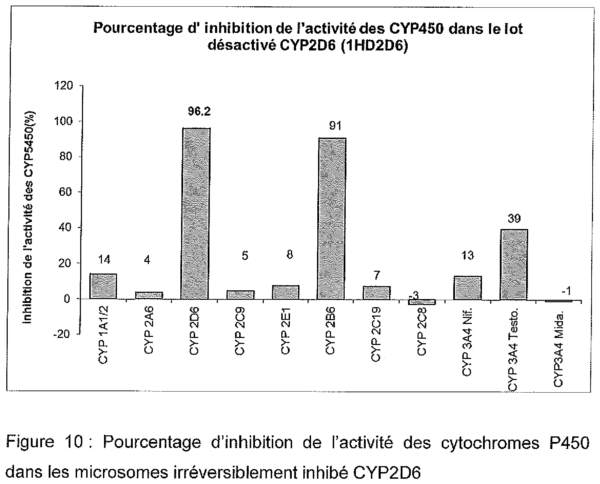

Figure 10 : Pourcentage d'inhibition de l'activité des cytochromes P450 dans les microsomes irréversiblement inhibé CYP2D6. Les activités spécifiques étudiées pour chaque CYP450 correspondent à celles décrites à la Figure 1. Le pourcentage d'inhibition est obtenu par comparaison des activités P450 sur microsomes inhibés irréversiblement par la paroxetine et sur les microsomes témoins.

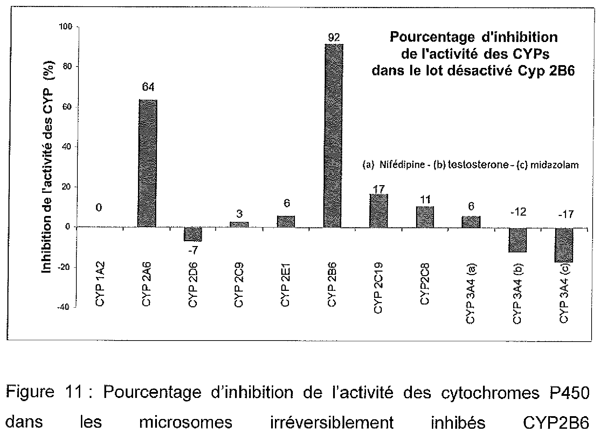

Figure 11 : Pourcentage d'inhibition de l'activité des cytochromes P450 dans les microsomes irréversiblement inhibés CYP2B6.
   Les activités spécifiques étudiées pour chaque CYP450 correspondent aux activités de phénacétine-O-déacéthylase (CYP1A2, incubation de phénacétine à 200 µM), coumarin-7-hydroxylase (CYP2A6, incubation de coumarine à 20 µM), bupropion-hydroxylase (2B6, incubation de bupropion à 100 µM), amodiaquine-desethylase (2C8, incubation d'amodiaquine à 20 µM), diclofenac-4'-hydroxylase (2C9, incubation de diclofenac à 200 µM), S-mephenytoine-hydroxylase (2C19, incubation de S-mephenytoine à 60 µM), dextromethorphan-O-déméthylase (2D6, incubation de dextromethorphan à 100 µM), chlorzoxazone-6-hydroxylase (2E1, incubation de chlorzoxazone à 200 µM) et testostérone-6β-hydroxylase, midazolam-1'-hydroxylase et nifédipine-réductase (3A4, incubation de testostérone à 75 µM, midazolam à 50 µM, nifédipine à 50 µM).
   Le pourcentage d'inhibition est obtenu par comparaison des activités P450 sur microsomes inhibés irréversiblement par le thioTEPA et sur les microsomes témoins.

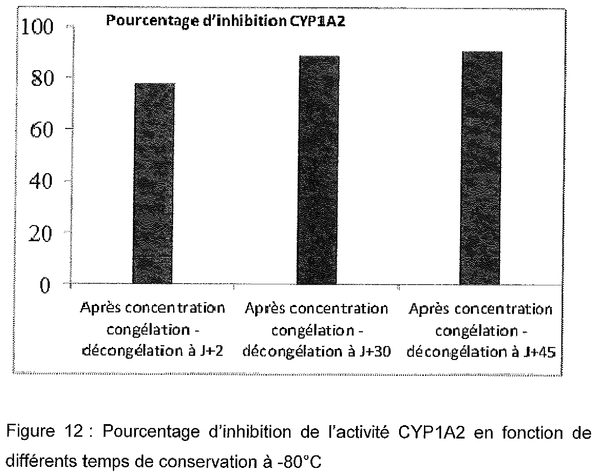

Figure 12 : Pourcentage d'inhibition de l'activité CYP1A2 en fonction de différents temps de conservation à -80°C.

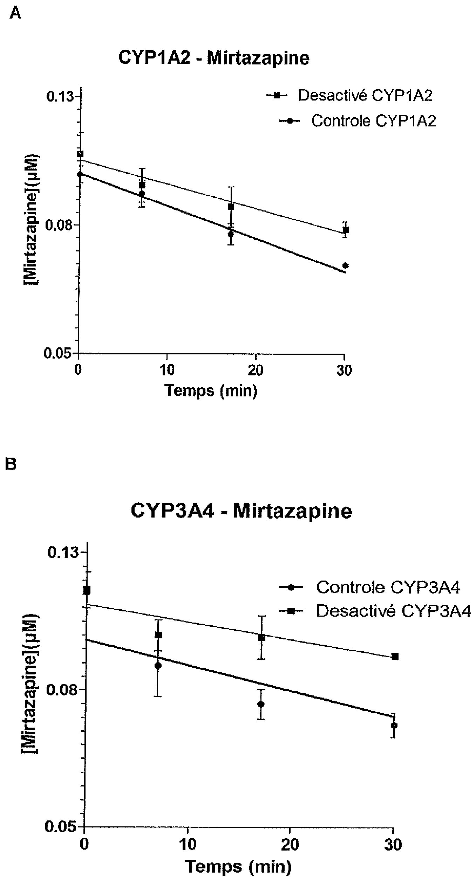

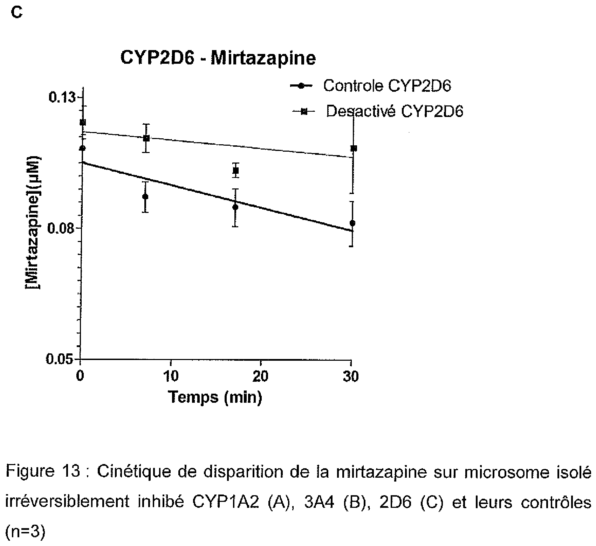

Figure 13 : Cinétique de disparition de la mirtazapine sur microsome isolé irréversiblement inhibé CYP1A2 (A), 3A4 (B), 2D6 (C) et leurs contrôles (n=3).

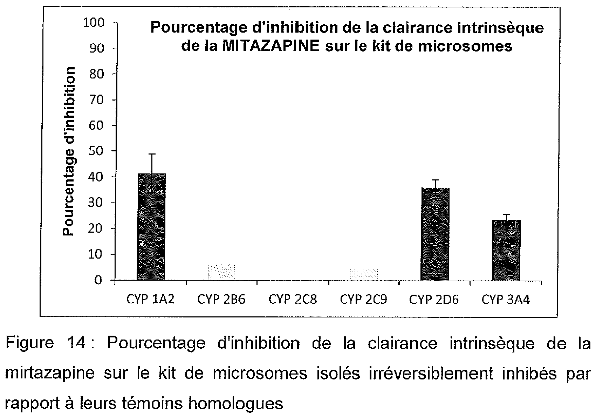

Figure 14 : Pourcentage d'inhibition de la clairance intrinsèque de la mirtazapine sur le kit de microsomes isolés irréversiblement inhibés par rapport à leurs témoins homologues.

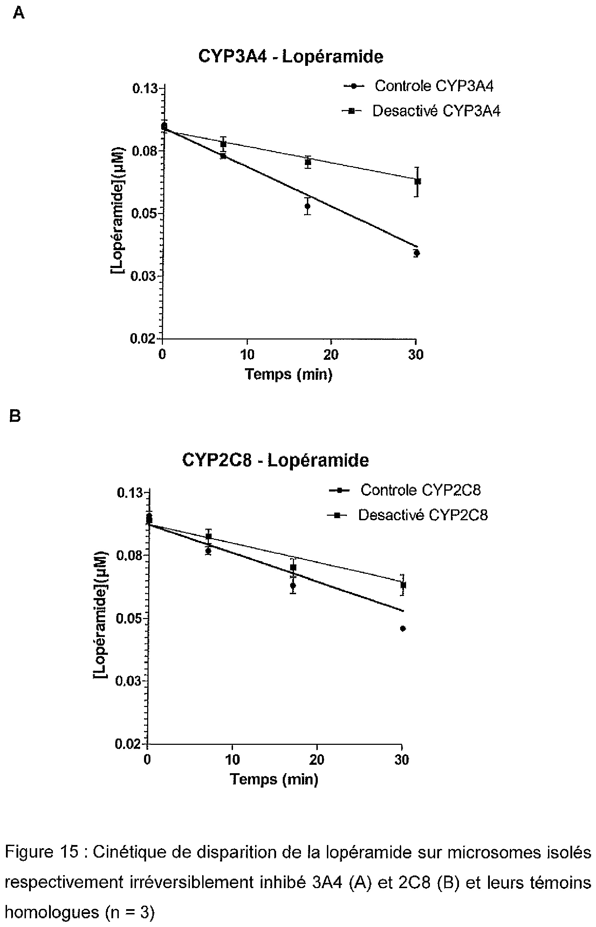

Figure 15 : Cinétique de disparition de la lopéramide sur microsomes isolés respectivement irréversiblement inhibé 3A4 (A) et 2C8 (B) et leurs témoins homologues (n = 3).

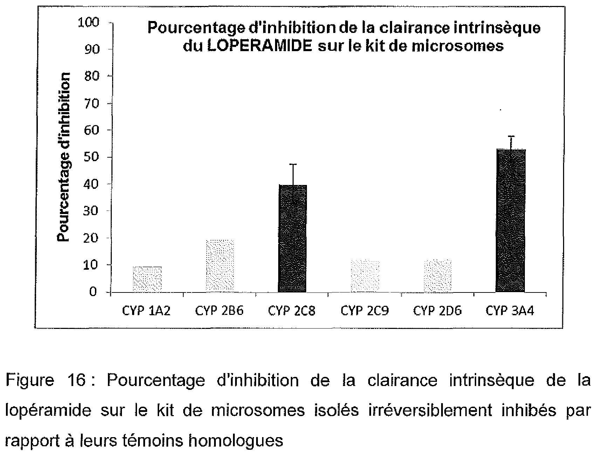

Figure 16: Pourcentage d'inhibition de la clairance intrinsèque de la lopéramide sur le kit de microsomes isolés irréversiblement inhibés par rapport à leurs témoins homologues.

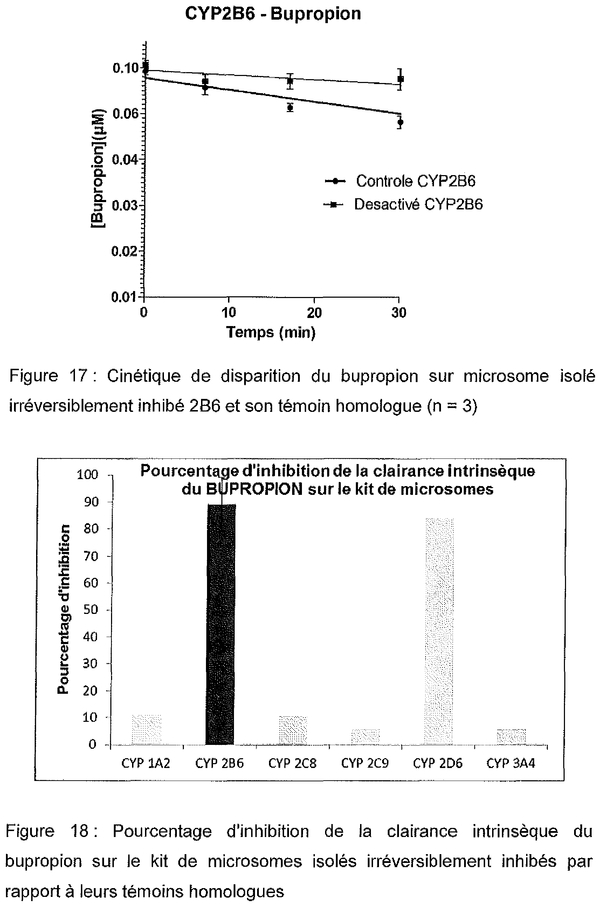

Figure 17 : Cinétique de disparition du bupropion sur microsome isolé irréversiblement inhibé 2B6 et son témoin homologue (n = 3).
Figure 18 : Pourcentage d'inhibition de la clairance intrinsèque du bupropion sur le kit de microsomes isolés irréversiblement inhibés par rapport à leurs témoins homologues.

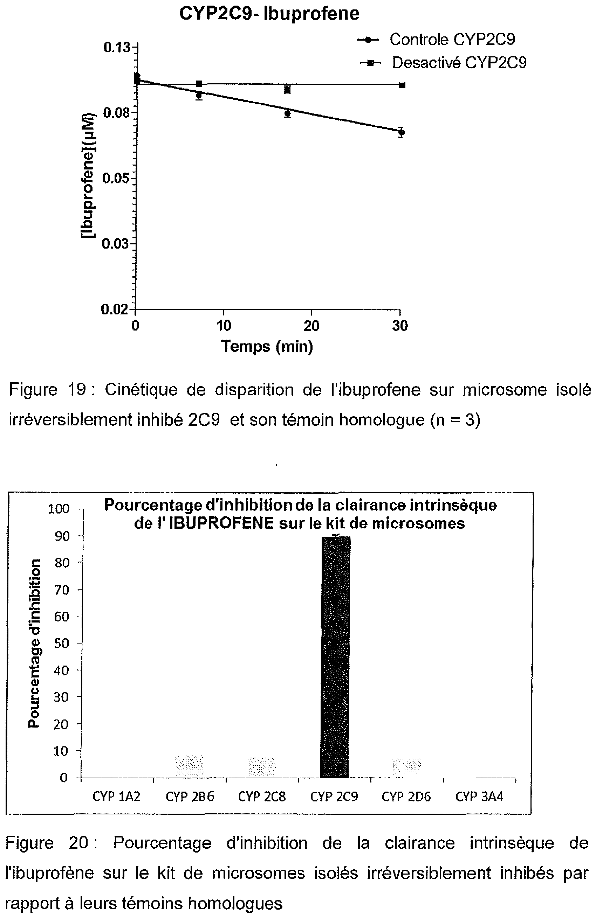

Figure 19 : Cinétique de disparition de l'ibuprofene sur microsome isolé irréversiblement inhibé 2C9 et son témoin homologue (n = 3).
Figure 20 : Pourcentage d'inhibition de la clairance intrinsèque de l'ibuprofène sur le kit de microsomes isolés irréversiblement inhibés par rapport à leurs témoins homologues.

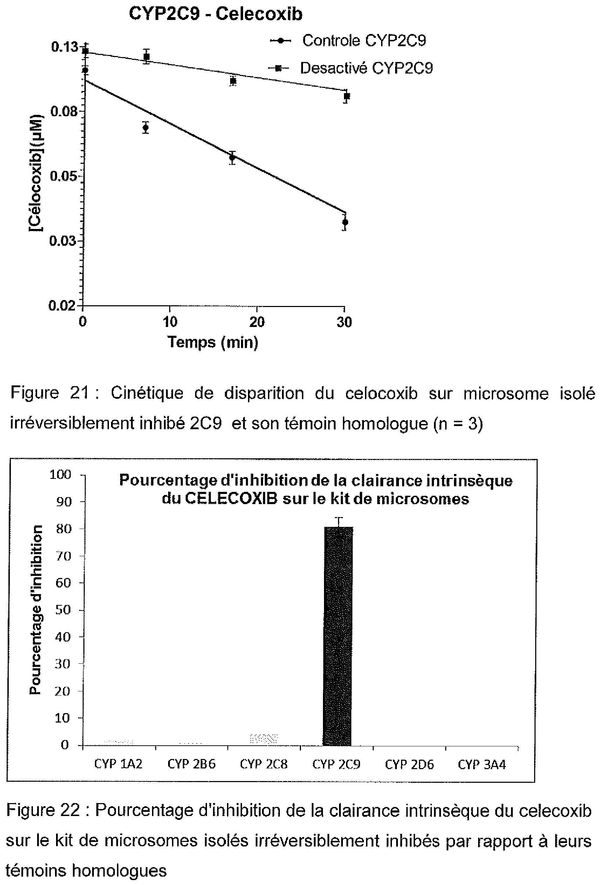

Figure 21 : Cinétique de disparition du celocoxib sur microsome isolé irréversiblement inhibé 2C9 et son témoin homologue (n = 3).
Figure 22 : Pourcentage d'inhibition de la clairance intrinsèque du celecoxib sur le kit de microsomes isolés irréversiblement inhibés par rapport à leurs témoins homologues.

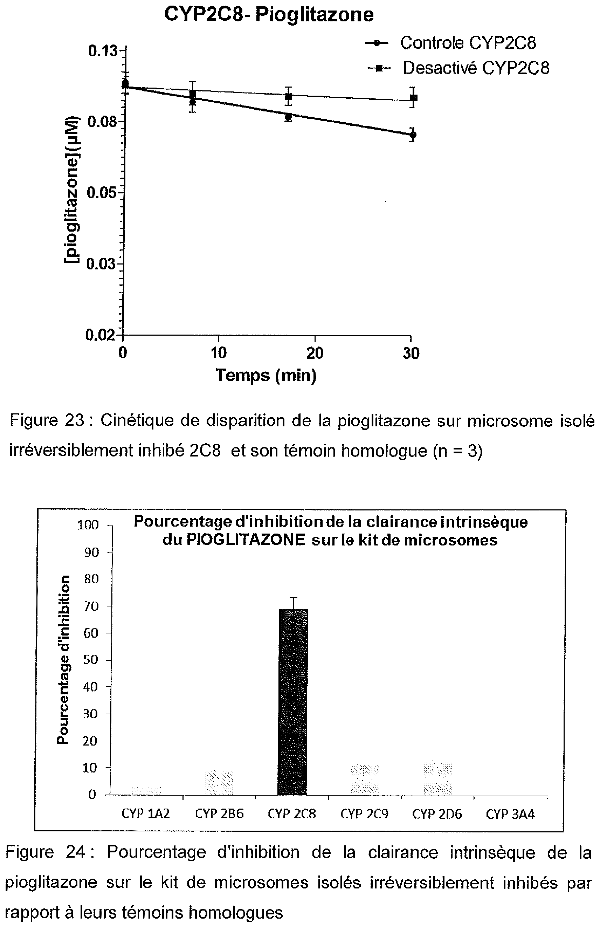

Figure 23 : Cinétique de disparition de la pioglitazone sur microsome isolé irréversiblement inhibé 2C8 et son témoin homologue (n = 3).
Figure 24 : Pourcentage d'inhibition de la clairance intrinsèque de la pioglitazone sur le kit de microsomes isolés irréversiblement inhibés par rapport à leurs témoins homologues.

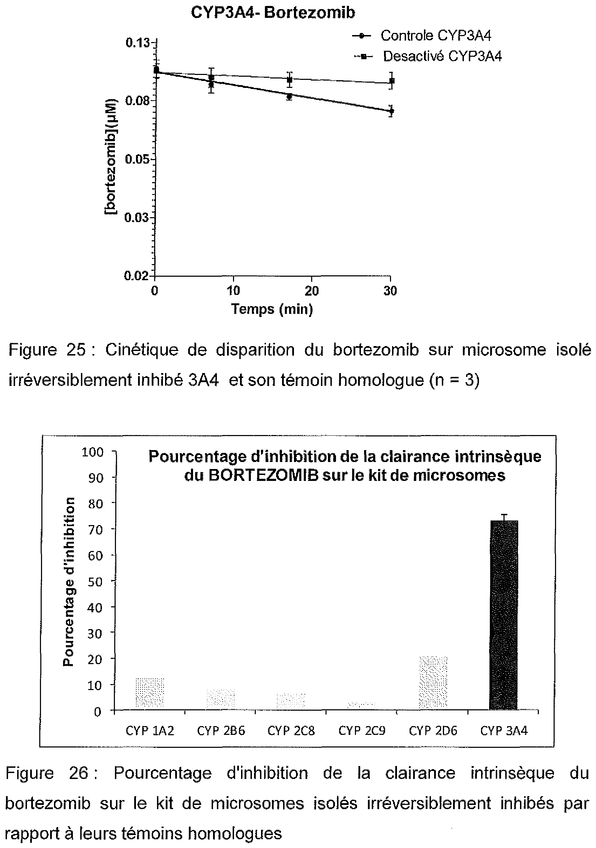

Figure 25 : Cinétique de disparition du bortezomib sur microsome isolé irréversiblement inhibé 3A4 et son témoin homologue (n = 3).
Figure 26 : Pourcentage d'inhibition de la clairance intrinsèque du bortezomib sur le kit de microsomes isolés irréversiblement inhibés par rapport à leurs témoins homologues.

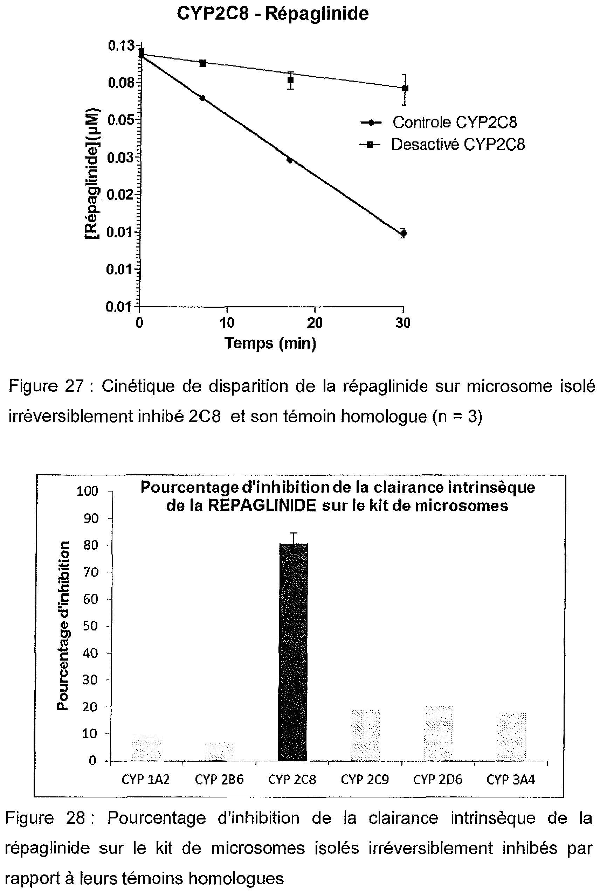

Figure 27 : Cinétique de disparition de la répaglinide sur microsome isolé irréversiblement inhibé 2C8 et son témoin homologue (n = 3).
Figure 28 : Pourcentage d'inhibition de la clairance intrinsèque de la répaglinide sur le kit de microsomes isolés irréversiblement inhibés par rapport à leurs témoins homologues.

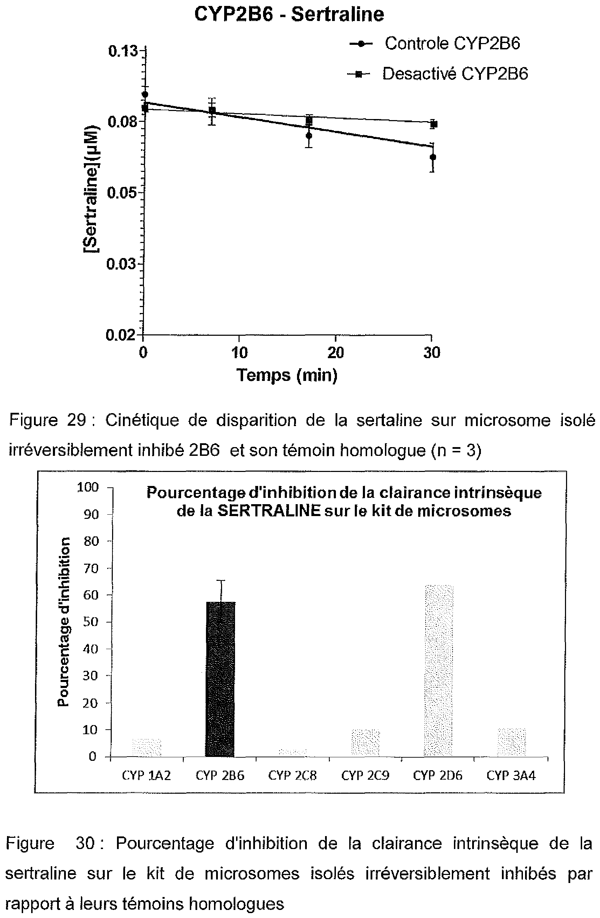

Figure 29 : Cinétique de disparition de la sertaline sur microsome isolé irréversiblement inhibé 2B6 et son témoin homologue (n = 3).
Figure 30 : Pourcentage d'inhibition de la clairance intrinsèque de la sertraline sur le kit de microsomes isolés irréversiblement inhibés par rapport à leurs témoins homologues.

### Exemple 1 : préparation de microsomes isolés irréversiblement inactivés

### • Matériel biologique

Les microsomes sont issus de foies humains contenant l'ensemble des enzymes P450. Ils proviennent d'un pool de microsomes puisqu'ils sont issus de plusieurs donneurs afin de tenir compte de la variabilité interindividuelle.

### • Incubation des microsomes

Pour chaque préparation de lot de microsomes irréversiblement inhibés, un lot témoin est préparé dans les mêmes conditions à la différence que l'inhibiteur irréversible est remplacé par un volume équivalent de solvant.

L'inhibiteur non réversible du cytochrome P450 étudié (ou le solvant, pour le lot témoin) est incubé avec les microsomes dans du tampon Tris/HCl à pH 7.4 et du MgCl₂ sous agitation à 37°C. La préparation est généralement préchauffée entre 5 et 10 minutes puis on ajoute du NADPH pour démarrer la réaction enzymatique. Au temps t, le milieu réactionnel est placé pendant quelques minutes dans la glace avant de passer à l'étape de concentration. Des complexes enzyme-inhibiteur liés par liaisons covalentes sont formés au cours de l'incubation des microsomes avec l'inhibiteur irréversible. Le cytochrome P450 étudié est inhibé de manière irréversible, totale et spécifique.

### • Filtration et concentration des microsomes irréversiblement désactivés

L'échantillon issu de l'incubation des microsomes avec l'inhibiteur non réversible du cytochrome P450 étudié est filtré. Cette étape de filtration des protéines peut être réalisée grâce à une membrane avec un seuil de coupure de 10 000 à 40 000 Daltons. Un système Centricon® est utilisé pour effectuer cette étape de filtration.

Une ou plusieurs étapes de lavage sont parfois nécessaire pour faciliter l'élimination de l'inhibiteur irréversible restant libre. L'échantillon est centrifugé entre 3000 g à 4000 g pendant 80 minutes puis 800 à 1000 g pendant 5 minutes. L'échantillon peut subir une succession de centrifugations afin d'optimiser la concentration des protéines microsomales.

Le cas échéant, l'échantillon de microsomes concentré est ensuite ultracentrifugé afin d'améliorer encore la concentration des protéines. L'ultracentrifugation est réalisée dans une gamme de conditions comprises entre 80 000 g pendant 4 heures à 150 000g pendant 45 minutes, préférentiellement à 100 000 g pendant 1 heure.

Le concentrat de protéines est repris dans un tampon Tris/HCI à pH 7.4 puis aliquoté et congelé à -80°C.

A l'issue de cette préparation, sont obtenus des microsomes isolés irréversiblement inactivés utilisables de manière *ex temporané* pour le phénotypage des réactions enzymatiques impliquées dans le métabolisme d'un principe actif.

### Exemple 2 : Conditions d'inhibition des principaux cytochromes P450 et validation des microsomes irréversiblement inhibés sur des substrats spécifiques des cytochromes P450

### • CYP1A2

La furafylline est un des inhibiteurs MBI du cytochrome CYP1A2.

Les conditions expérimentales pour une inhibition MBI maximale par la furafylline sur le CYP1A2 sont les suivantes :
- protéines microsomales à 2 mg/ml ;
- furafylline à 10 µM ;
- temps de pré-incubation de 30 minutes.

Après incubation de la phénacétine, substrat spécifique du CYP1A2, à 4.5 µM (concentration inférieure ou égale à son Km) avec des microsomes préalablement inhibés selon les conditions ci-dessus, le pourcentage d'inhibition de l'activité phénacétine déacethylase (CYP1A1/CYP1A2 dépendante) est de 83% (Figure 1). Notons que les 17% de métabolisme restant sont dû à l'activité phénacétine déacethylase résiduelle liée au CYP1A1 et non à un défaut d'inhibition du CYP1A2. En effet il est connu que la phénacétine incubée dans des conditions non saturantes (< 5 µM) est métabolisée majoritairement par le CYP1A2 et partiellement par le CYP1A1.

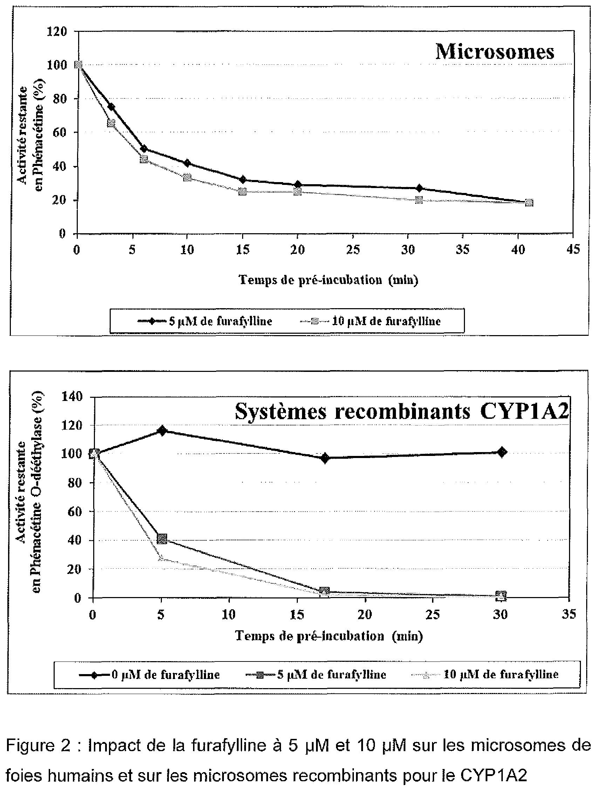

La furafylline préincubée pendant 30 minutes de 5 µM à 10 µM avec des CYP1A2 recombinants humains entraîne 100% d'inhibition de l'activité purement CYP1A2 de la phénacétine prouvant ainsi sa puissance maximale d'inhibition aux conditions retenues (Figure 2).

Lorsque la phénacétine est incubée à une concentration très supérieure à son Km pour le CYP1A2 en présence des microsomes irréversiblement inhibés et du lot témoin préparés selon les conditions ci-dessus, le pourcentage d'inhibition de l'activité phénacétine déacéthylase (CYP1A1/CYP1A2 dépendante) est toujours d'environ 80%. Ce résultat prouve que l'inhibition du CYP1A2 par la furafylline n'est pas affectée par un excès de substrat et qu'aucune inhibition de type compétitive n'est détectable.

Les substrats spécifiques des autres CYP450 majoritaires (CYP2A6, 2B6, 2C8, 2C9, 2C19, 2D6, 2E1, et 3A4) ont été incubés en présence du lot de microsomes de foies humains irréversiblement inhibés par la furafylline et du lot témoin dans les conditions définies ci-dessus afin de démontrer la spécificité de la furafylline. On observe que l'activité des autres CYP450 majoritaires reste inchangée.

L'ensemble de ces résultats montrent que les microsomes isolés et irréversiblement et spécifiquement inhibés vis-à-vis du CYP1A2 selon l'invention peuvent valablement être utilisés pour mesurer la contribution du CYP1A2 dans le métabolisme d'un principe actif ou d'un candidat médicament.

### • CYP3A4

L'azamulin est un des inhibiteurs MBI du cytochrome CYP3A4.

Les conditions expérimentales pour une inhibition MBI maximale par l'azamulin sur le CYP3A4 sont les suivantes :
- protéines microsomales à 2 mg/ml ;
- azamulin à 5 µM ;
- temps de pré-incubation de 15 minutes.

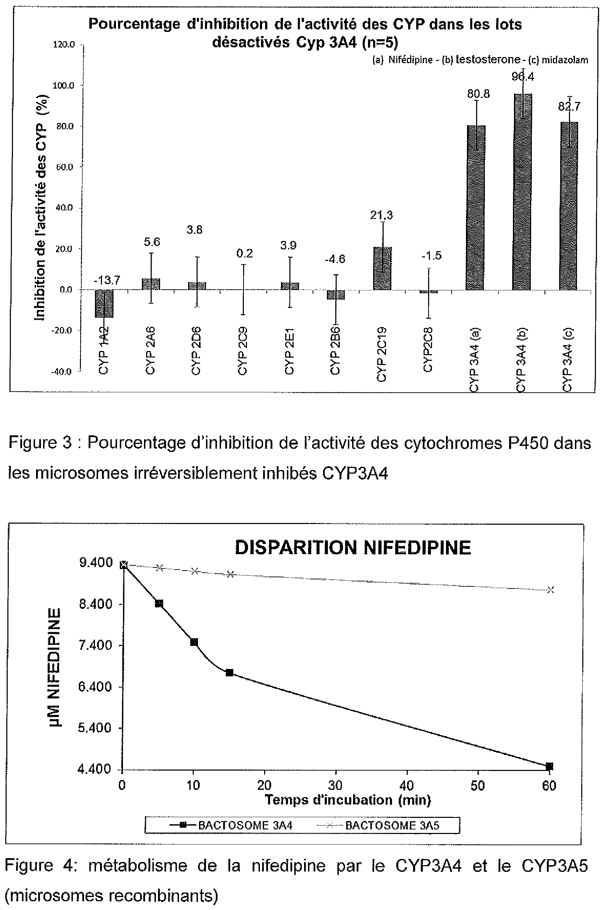

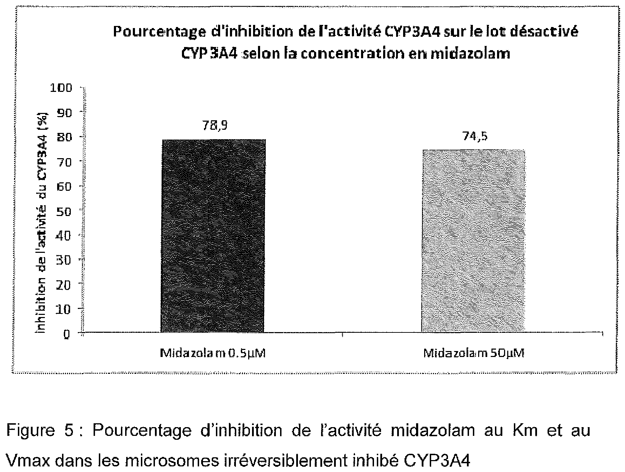

Après incubation du midazolam à 0.5 µM, de la testostérone à 30 µM et de la nifédipine à 10 µM (concentrations inférieures ou égales au Km des substrats), substrats spécifiques du CYP3A4, en présence des microsomes irréversiblement inhibés et du lot témoin préparés selon les conditions ci-dessus détaillées, les pourcentages d'inhibition des activités midazolam-1'-hydroxylase, testosterone-6β-hydroxylase, et nifédipine-réductase (CYP3A4/CYP3A5 dépendantes) sont respectivement de 81%, 96% et 83% (Figure 3). Notons que les 19%, 4%, 17% de métabolisme restant pour chacune de ces activités sont liés à l'activité CYP3A5 et non à un défaut d'inhibition du CYP3A4. En effet il est connu que les trois substrats spécifiques (et plus particulièrement nifedipine et midazolam) sont majoritairement métabolisés par le CYP3A4 mais également par le CYP3A5. La figure 4 montre notamment le métabolisme de la nifédipine par des microsomes recombinants (bactosomes) CYP3A4 et CYP3A5. L'azamulin pré-incubée pendant 15 minutes à 5 µM avec des CYP3A4 recombinants humains entraîne 92 % d'inhibition de l'activité purement CYP3A4 du midazolam prouvant sa puissance maximale d'inhibition aux conditions retenues. Lorsque les substrats spécifiques du CYP3A4 (exemple du midazolam) sont incubés à une concentration très supérieure à leur Km pour le CYP3A4 avec des microsomes préalablement préparés selon la présente invention, le pourcentage d'inhibition des activités CYP3A4 dépendantes reste inchangé (Figure 5). Ce résultat prouve que l'inhibition du CYP3A4 par l'azamulin n'est pas affectée par un excès de substrat et qu'aucune inhibition de type compétitive n'est détectable.

Les substrats spécifiques des autres CYP450 majoritaires (CYP1A2, CYP2A6, 2B6, 2C8, 2C9, 2C19, 2D6, et 2E1) ont été incubés en présence du lot de microsomes de foies humains irréversiblement inhibés par l'azamulin et du lot témoin dans les conditions définies ci-dessus afin de démontrer la spécificité de l'azamulin. La figure 3 montre que l'activité des autres CYP450 majoritaires reste inchangée entre le lot de microsomes humains irréversiblement inhibés et le lot témoin démontrant que l'azamulin est bien spécifique de l'activité CYP3A4.

L'ensemble de ces résultats montrent que les microsomes isolés et irréversiblement inhibés vis-à-vis du CYP3A4 selon l'invention peuvent valablement être utilisés pour mesurer la contribution du CYP3A4 dans le métabolisme d'un principe actif ou d'un candidat médicament.

### • CYP2C8

Le gemfibrozil glucuronide est un des inhibiteurs MBI du cytochrome CYP2C8. Les conditions expérimentales pour une inhibition MBI maximale par le gemfibrozil glucuronide sur le CYP2C8 sont les suivantes :
- protéines microsomales à 2 mg/ml ;
- gemfibrozil glucuronide à 30 µM ;
- temps de pré-incubation de 30 minutes.

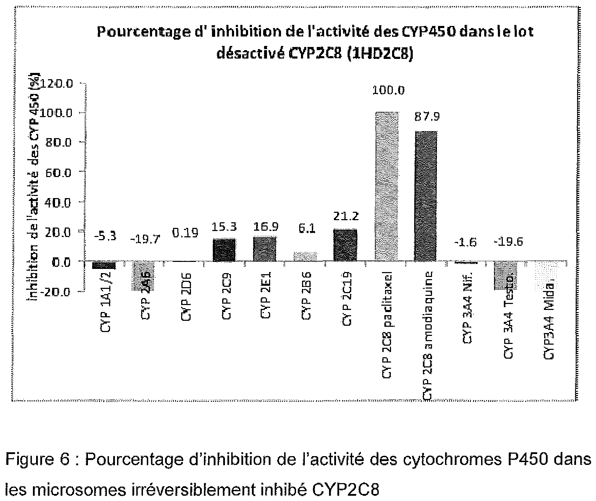

Après incubation de l'amodiaquine à 0.5 µM ou du paclitaxel à 4 µM (concentrations inférieures ou égales au Km des deux substrats pour le CYP2C8), substrats spécifiques du CYP2C8, en présence des microsomes irréversiblement inhibés et du lot témoin préparés selon la présente invention, le pourcentage d'inhibition des activités amodiaquine (CYP2C8) et paclitaxel-hydroxylase (CYP2C8 dépendante) est respectivement de 88% et 100% (Figure 6).

Lorsque l'amodiaquine est incubée à une concentration très supérieure à son Km pour le CYP2C8 dans les mêmes conditions que celles décrites supra, le pourcentage d'inhibition de l'activité amodiaquine hydroxylase reste inchangée (Figure 7). Ce résultat prouve que l'inhibition du CYP2C8 par l'amodiaquine n'est pas affectée par un excès de substrat et qu'aucune inhibition de type compétitive n'est détectable.

Les substrats spécifiques des autres CYP450 majoritaires ont été incubés dans les conditions définies ci-dessus afin de démontrer la spécificité de la gemfibrozil glucuronide. La figure 6 montre que l'activité des autres CYP450 majoritaires reste inchangée à l'exception d'une légère inhibition du CYP2C19 entre le lot de microsomes humains irréversiblement inhibé et le lot témoin démontrant que le gemfibrozil glucuronide est bien spécifique de l'activité CYP2C8.

L'ensemble de ces résultats montrent que les microsomes isolés et irréversiblement inhibés vis-à-vis de l'activité CYP2C8 selon l'invention peuvent valablement être utilisés pour mesurer la contribution du CYP2C8 dans le métabolisme d'un principe actif ou d'un candidat médicament.

### • CYP2C9

L'acide tienilique est un des inhibiteurs MBI du cytochrome CYP2C9.

Les conditions expérimentales pour une inhibition MBI maximale par l'acide tienilique sur le CYP2C9 sont les suivantes :
- protéines microsomales à 2 mg/ml ;
- acide tienilique à 10 µM ;
- temps de pré-incubation de 20 minutes.

Après incubation du diclofenac, substrat spécifique du CYP2C9, à 4 µM (concentration inférieure ou égale au Km du substrat pour le CYP2C9) et 100 µM (concentration très supérieure à son Km pour le CYP2C9) en présence des microsomes irréversiblement inhibé et du lot témoin préparés selon la présente invention, l'inhibition de l'activité diclofenac hydroxylase (CYP2C9 dépendante) est quasi-totale soit respectivement 92% et 88% d'inhibition (Figure 8). Ce résultat prouve que non seulement l'inhibition du CYP2C9 est totale mais aussi qu'elle n'est pas affectée par un excès de substrat et qu'aucune inhibition de type compétitive n'est détectable.

Les substrats spécifiques des autres CYP450 majoritaires ont été incubés dans les conditions définies ci-dessus afin de démontrer la spécificité de l'acide tienilique. La figure 9 montre que l'activité des autres CYP450 majoritaires reste inchangée entre le lot de microsomes irréversiblement inhibé et le lot témoin démontrant que l'acide tienilique est bien spécifique de l'activité CYP2C9.

L'ensemble de ces résultats montrent que les microsomes isolés et irréversiblement inhibés vis-à-vis de l'activité CYP2C9 selon l'invention peuvent valablement être utilisés pour mesurer la contribution du CYP2C9 dans le métabolisme d'un principe actif ou d'un candidat médicament.

### • CYP2D6

La paroxetine est un des inhibiteurs MBI du cytochrome CYP2D6.

Les conditions expérimentales pour une inhibition MBI maximale par la paroxetine sur le CYP2D6 sont les suivantes :
- protéines microsomales à 2mg/ml ;
- paroxetine à 50 µM ;
- temps de pré-incubation de 30 minutes.

Après incubation du dextromethorphan, substrat spécifique du CYP2D6, à 5 µM (concentration inférieure ou égale au Km du substrat) et 50 µM (concentration très supérieure à son Km pour le CYP2D6) en présence des microsomes irréversiblement inhibé et du lot témoin préparés selon la présente invention, l'inhibition de l'activité dextromethorphan-O-demethylase (CYP2D6 dépendante) est quasi-totale soit 96% d'inhibition (Figure 10). Ce résultat prouve que l'inhibition du CYP2D6 est totale, qu'elle n'est pas affectée par un excès de substrat et qu'aucune inhibition de type compétitive n'est détectable.

Les substrats spécifiques des autres CYP450 majoritaires ont été incubés dans les conditions définies ci-dessus afin de démontrer la spécificité de la paroxetine. La figure 10 montre que parmi toutes les autres activités CYP450, seule l'activité bupropion hydroxylase dépendante du CYP2B6 est inhibée à 91% en supplément du CYP2D6 démontrant que la paroxetine n'est pas totalement spécifique de l'activité CYP2D6.

L'ensemble de ces résultats montrent que des microsomes préparés selon l'invention permettent d'inhiber totalement et quasi spécifiquement l'activité CYP2D6. Ils peuvent donc être utilisés pour mesurer la contribution du CYP2D6/CYP2B6 dans le métabolisme d'un nouveau candidat médicament.

### • CYP2B6

Le thioTEPA est un des inhibiteurs MBI du cytochrome CYP2B6.

Les conditions expérimentales pour une inhibition MBI maximale par le thioTEPA sur le CYP2B6 sont les suivantes :
- protéines microsomales à 2mg/ml ;
- thioTEPA à 15 µM ;
- temps de pré-incubation de 30 minutes.

Après incubation du bupropion, substrat spécifique du CYP2B6, à 100 µM en présence des microsomes irréversiblement inhibés et du lot témoin préparé selon la présente invention, l'inhibition de l'activité bupropion hydroxylase (CYP2B6 dépendante) est quasi-totale soit 92% d'inhibition (Figure 11). Ce résultat prouve que l'inhibition du CYP2B6 est totale, qu'elle n'est pas affectée par un excès de substrat et qu'aucune inhibition de type compétitive n'est détectable.

Les substrats spécifiques des autres CYP450 majoritaires ont été incubés dans les conditions définies ci-dessus afin de démontrer la spécificité du thioTEPA. La figure 11 montre que parmi toutes les autres activités CYP450, seule l'activité coumarine hydroxylase dépendante du CYP2A6 est inhibée à 64% en supplément du CYP2B6 démontrant que le thioTEPA n'est pas totalement spécifique de l'activité CYP2B6.

L'ensemble de ces résultats montrent que des microsomes préparés selon l'invention permettent d'inhiber totalement et quasi spécifiquement l'activité CYP2B6. Ils peuvent donc être utilisés pour mesurer la contribution du CYP2B6/CYP2A6 dans le métabolisme d'un nouveau candidat médicament.

### Exemple 3 : Stabilité de l'inhibition des cytochromes P450 dans les microsomes isolés, irréversiblement inhibés et conservés par congélation

Des microsomes isolés de foies humains irréversiblement inhibés en CYP1A2 ayant été concentrés et conservés à -80°C conformément à l'invention sont incubés pendant 15 minutes avec de la phénacétine (4.5µM) substrat spécifique de CYP1A2 à 1mg/mL. Les pourcentages d'inhibition ont été mesurés pour des microsomes obtenus selon l'invention et conservés à -80°C pendant 48 heures, un mois et un mois et demi (Figure 12). On observe que les étapes de concentration, congélation-décongélation n'altèrent pas l'inhibition MBI du CYP1A2 par la furafylline. Les étapes de congélation et décongélation n'ont pas d'influence sur la stabilité de l'inhibition irréversible des cytochromes P450.

### Exemple 4: Kit de microsomes isolés irréversiblement inhibés pour le phénotypage enzymatique des voies métaboliques de xénobiotiques

Neuf principes actifs (mirtazapine, loperamide, bupropion, ibuprofène, celecoxib, pioglitazone, bortezomib, repaglinide, sertraline) ont été testés dans un kit de microsomes isolés irréversiblement inhibés selon l'invention pour le phénotypage enzymatique des voies métaboliques desdits neuf xénobiotiques. Les neuf principes actifs ont été testés selon la méthode de phénotypage des réactions enzymatiques selon l'invention et comprenant les étapes suivantes :
- l'incubation de microsomes isolés et irréversiblement inhibés selon l'invention avec un principe actif à évaluer ;
- la mesure de la contribution du cytochrome P450 irréversiblement inhibé impliqué dans le métabolisme du principe actif.

Chaque principe actif a été incubé à 0.1 µM à 37 °C dans du tampon Tris/HCl (0.1mM, pH 7.4) additionné de MgCl₂ 5 mM, sur d'une part les microsomes de foie isolés, irréversiblement inhibés pour les CYP450 1A2, 2B6, 2C8, 2C9, 2D6 et 3A4 et d'autre part sur les microsomes de foie isolés, non inhibés et préparés selon l'invention (témoin homologue). La réaction est initiée par l'ajout de NADPH 1 mM. L'incubation est suivie sous-forme d'une cinétique. Aux temps d'incubation à 7 min, 17 min, 30 min puis 60 min, une aliquote d'incubation (100 µL) est prélevée et la réaction enzymatique est arrêtée en ajoutant à cette aliquote un volume de solvant (100 µL de méthanol) laquelle est placée dans la glace pendant 10 minutes.

A chaque temps d'incubation, le principe actif est quantifié par chromatographie liquide haute performance (HPLC) couplée à la spectrométrie de masse (MS).

L'activité métabolique (A) du principe actif est mesurée via la clairance intrinsèque métabolique du principe actif inchangé dans les deux conditions, inhibée (activité Ai) et non inhibée (témoin avec l'activité A).

Le pourcentage d'inhibition calculé (pourcentage d'inhibition = (A-Ai)/A) correspond directement à la contribution du cytochrome P450 irréversiblement inhibé impliqué dans le métabolisme du principe actif.

### • Mirtazapine

La mirtazapine a été incubée selon les conditions décrites précédemment avec une concentration en protéines microsomales de 2 mg/ml permettant une mesure optimale de sa clairance intrinsèque. En présence de microsomes témoins (non inhibés et préparés selon l'invention), une clairance intrinsèque de 3.9 à 7.8 ml/min/g protéines a été mesurée. Par rapport aux microsomes témoin, une inhibition de la clairance intrinsèque de la mirtazapine de 41%, 36% et 24% a été mise en évidence en présence des microsomes de foie isolés irréversiblement inhibés respectivement pour les CYP450 1A2, 2D6 et 3A4 (Figures 13 et 14). Aucune inhibition significative de la clairance intrinsèque de la mirtazapine n'a été observée en présence des microsomes de foie isolés irréversiblement inhibés pour les CYP450 2B6, 2C8, 2C9. Par inhibition significative, on entend une clairance intrinsèque inférieure à 25%, pourcentage représentant le seuil de variabilité observée sur des mesures de clairance sur microsomes de foie. En conséquence, le métabolisme oxydatif de la mirtazapine implique les CYP450 1A2, 2D6 et 3A4 respectivement à hauteur de 41%, 36% et 24%.

Après incubation de la mirtazapine de 2.5 à 1000 µM en présence de microsomes recombinant surexprimant les CYP450 humains majoritaires et après avoir mesuré le facteur de correction adapté pour chacun de ces CYP450, Störmer et al. (Metabolism of the antidepressant mirtazapine in vitro: contribution of cytochromes P-450 1A2, 2D6 and 3A4. Drug Metab Dispos. 2000; 28(10): 1168-1175) ont montré que les CYP450 1A2, 2D6 et 3A4 étaient respectivement impliqués à 41%, 39% et 23% dans le métabolisme de la mirtazapine. Les résultats obtenus avec le kit de microsomes isolés irréversiblement inhibés selon l'invention sont corroborés par les résultats obtenus par Störmer et al. (Tableau 1).

Le kit selon la présente invention permet de déduire l'implication des CYP450 dans le métabolisme oxydatif de la mirtazapine par simple comparaison des clairances intrinsèques en présence de microsomes de foie isolés, irréversiblement inhibés pour les CYP450 et de microsomes témoins.

En revanche, l'utilisation de microsomes recombinant surexprimant les CYP450 humains pour le phénotypage des voies enzymatiques de la mirtazapine exige une mesure indirecte nécessitant de caractériser chaque activité CYP450 d'une part sur mirtazapine et d'autre part sur des substrats spécifiques dans un premier temps sur les microsomes recombinants surexprimant les CYP450 humains et dans un second temps sur les microsomes de foies humains afin de mesurer le facteur de correction.

**Tableau 1 : Pourcentage d'implication des CYP450 dans le métabolisme oxydatif de la mirtazapine obtenus à partir du kit de microsomes isolés irréversiblement inhibés selon l'invention et avec des enzymes recombinantes humaines (Stôrmer et al.)**

| CYP450 impliqué | % d'implication des CYP450 dans le métabolisme oxydatif de la Mirtazapine | |
|---|---|---|
| | Kit de microsomes isolés | Données attendues* |
| CYP1A2 | 41 | 41 |
| CYP2D6 | 36 | 39 |
| CYP3A4 | 24 | 23 |

| | | |
|---|---|---|
| * de Störmer et al (2000) sur modèle d'enzymes humaines recombinantes | | |

### • Loperamide

La loperamide a été incubée selon les conditions décrites précédemment avec une concentration en proteines microsomales de 2 mg/ml permettant une mesure optimale de sa clairance intrinsèque. En présence de microsomes témoins (non inhibés et préparés selon l'invention), une clairance intrinsèque de 14.5 à 17.2 ml/min/g protéines a été mesurée. Par rapport aux microsomes témoins, une inhibition de la clairance intrinsèque de la loperamide de 53% et 40% a été mise en évidence en présence des microsomes de foie isolés irréversiblement inhibés respectivement pour les CYP450 3A4 et 2C8 (Figures 15 et 16). Aucune inhibition significative de la clairance intrinsèque de la loperamide n'a été observée en présence des microsomes de foie isolés irréversiblement inhibés pour les CYP450 1A2, 2B6, 2C9, 2D6. Par inhibition significative, on entend une clairance intrinsèque inférieure à 25%, pourcentage représentant le seuil de variabilité observée sur des mesures de clairance sur microsomes de foie. En conséquence, le métabolisme oxydatif de la loperamide implique les CYP450 3A4 et 2C8 respectivement à hauteur de 53% et 40%.

Une étude chez le volontaire sain montre que l'administration orale du gemfibrosil à 600 mg, inhibiteur du CYP2C8, augmente d'un facteur 2.2 l'exposition (AUC) en loperamide co-administrée per os à 4 mg (Niemi et al. Itraconazole, gemfibrozil and their combination markedly raise the plasma concentrations of loperamide. Eur J Clin Pharmacol. 2006; 62: 463-472). Cette augmentation de l'exposition correspond à une implication du CYP2C8 estimée à 55 % de la clairance totale de la loperamide. Dans cette même étude *in vivo,* une co-administration de la loperamide 4 mg avec 100 mg d'itraconazole, inhibiteur du CYP3A4, montre une augmentation de l'exposition d'un facteur 3.8 correspondant à environ 74% de la clairance totale de la loperamide.

Par ailleurs, Tayrouz et al. (Ritonavir increases loperamide plasma concentrations without evidence for P-glycoprotein involvement. Clin Pharmacol Ther. 2001 Nov;70(5):405-14) montrent chez le volontaire sain que la co-administration de la loperamide 16 mg avec 600 mg de ritonavir, inhibiteur du CYP3A4, induit une augmentation de l'exposition d'un facteur 2.65 correspondant à 62 % de la clairance totale de la loperamide.

Les résultats obtenus avec le kit de microsomes isolés irréversiblement inhibés selon l'invention (Tableau 2) sont corroborés par les données décrites en situation clinique par Niemi et al. et Tayrouz et al.

**Tableau 2 : Pourcentages d'implication des CYP450 dans le métabolisme oxydatif de la loperamide obtenus à partir du kit de microsomes isolés irréversiblement inhibés selon l'invention et chez le volontaire sain (Niemi et al. ; Tayrouz et al.)**

| CYP450 impliqué | % d'implication des CYP450 dans le métabolisme oxydatif de la Loperamide | | |
|---|---|---|---|
| | Kit de microsomes isolés | Données attendues¹ | Données attendues² |
| CYP2C8 | 40 | 55 | |
| CYP3A4 | 53 | 74 | 62 |

| | | | |
|---|---|---|---|
| ¹ de Niemi et al (2006) sur étude *in vivo* chez le volontaire sain ² de Tayrouz et al (2001) sur étude *in vivo* chez le volontaire sain | | | |

### • Bupropion

Le bupropion a été incubé selon les conditions décrites précédemment avec une concentration en protéines microsomales de 2 mg/ml permettant une mesure optimale de sa clairance intrinsèque. En présence des microsomes témoins (non inhibés et préparés selon l'invention), une clairance intrinsèque de 6.7 à 10.6 ml/min/g protéines a été mesurée. Par rapport aux microsomes témoins, une inhibition de la clairance intrinsèque du bupropion de 89% a été mise en évidence en présence des microsomes de foie isolés irréversiblement inhibés pour le CYP450 2B6 (Figures 17 et 18). Il est également observé une inhibition de la clairance intrinsèque du bupropion de 84% en présence des microsomes de foie isolés irréversiblement inhibés pour le CYP2D6. Sachant que la paroxetine, inhibiteur MBI du CYP2D6, n'est pas spécifique et inhibe également le CYP2B6, on en déduit donc que l'inhibition du CYP2D6 correspond en réalité à celle du CYP2B6.

Aucune inhibition significative de la clairance intrinsèque du bupropion n'a été observée en présence des microsomes de foie isolés irréversiblement inhibés pour les CYP450 1A2, 2C8, 2C9 et 3A4. Par inhibition significative, on entend une clairance intrinsèque inférieure à 25 %, pourcentage représentant le seuil de variabilité observée sur des mesures de clairance sur microsomes de foie. En conséquence, le métabolisme oxydatif du bupropion implique le CYP450 2B6 à hauteur de 89 %.

La FDA propose le bupropion comme substrat le plus sensible pour le CYP2B6 lors des études d'interaction *in vivo* chez l'homme (FDA Website on Drug Development and Drug Interactions, http://www.fda.gov/Drugs/GuidanceComplianceRegulatoryInformation/Guid ances/default.htm and http://www.fda.gov/Drugs/DevelopmentApprovalProcess/DevelopmentReso urces/DruginteractionsLabelin 1277 g/ucm080499.htm).

Les résultats obtenus avec le kit de microsomes isolés irréversiblement inhibés selon l'invention sont corroborés par les données décrites par la FDA.

**Tableau 3 : Pourcentage d'implication des CYP450 dans le métabolisme oxydatif du bupropion obtenu à partir du kit de microsomes isolés irréversiblement inhibés selon l'invention**

| CYP450 impliqué | % d'implication des CYP450 dans le métabolisme oxydatif du Bupropion | |
|---|---|---|
| | Kit de microsomes isolés | Données attendues* |
| CYP2B6 | 89 | majoritaire |

| | | |
|---|---|---|
| * de FDA (substrat sensible CYP2B6) | | |

### • Ibuprofene

L'ibuprofène a été incubé selon les conditions décrites précédemment avec une concentration en protéines microsomales de 0,25 mg/ml permettant une mesure optimale de sa clairance intrinsèque. En présence de microsomes témoin (non inhibés et préparés selon l'invention), une clairance intrinsèque de 31 à 54 ml/min/g protéines a été mesurée. Par rapport aux microsomes témoins, une inhibition de la clairance intrinsèque de l'ibuprofène de 90 % a été mise évidence en présence des microsomes de foie isolés irréversiblement inhibés pour le CYP450 2C9 (Figures 19 et 20).

Aucune inhibition significative de la clairance intrinsèque de l'ibuprofène n'a été observée en présence des microsomes de foie isolés irréversiblement inhibés pour les CYP450 1A2, 2B6, 2D6, 2C8 et 3A4. Par inhibition significative, on entend une clairance intrinsèque inférieure à 25%, pourcentage représentant le seuil de variabilité observée sur des mesures de clairance sur microsomes de foie. En conséquence, le métabolisme oxydatif de l'ibuprofène implique le CYP450 2C9 à hauteur de 90%.

Après incubation de l'ibuprofène à 3 µM en présence de microsomes recombinants sur exprimant les CYP450 humains majoritaires et après avoir mesuré le facteur de correction adapté pour chacun de ces CYP450, McGinnity et al. (Automated définition of the enzymology of drug oxidation by the major human drug metabolizing cytochrome P450s. Drug Metab Dispos. 2000 Nov;28(11): 1327-34) ont montré que le CYP450 2C9 était impliqué à 90% dans le métabolisme de l'ibuprofène. Les résultats obtenus avec le kit de microsomes isolés irréversiblement inhibés selon l'invention sont corroborés par les données décrites par McGinnity et al. (Tableau 4). Pour mémoire, le kit selon la présente invention établit l'implication des CYP450 dans le métabolisme oxydatif de l'ibuprofène par simple comparaison entre les clairances intrinsèques de microsomes selon l'invention et de microsomes témoins. En revanche, l'utilisation des microsomes recombinants imposent des mesures indirectes exigeant la multiplication des manipulations.

Par ailleurs, une étude chez le volontaire sain montre que l'administration orale du fluconazole à 400 mg, augmente de 83% l'exposition (AUC) en ibuprofène co-administré per os à 400 mg (Hynninen et al. Effects of the Antifungals Voriconazole and Fluconazole on the Pharmacokinetics of S-(+)- and R-(-)-Ibuprofen. Antimicrob Agents Chemother. Jun 2006; 50(6): 1967-1972). Lazar et al. (Drug interactions with fluconazole. Rev Infect Dis. 1990 Mar-Apr;12 Suppl 3:S327-33) ont montré que le fluconazole, inhibiteur du CYP2C9, entraine une augmentation de l'exposition de 109 % de la tolbutamide substrat reconnu comme sensible pour le CYP2C9 (fm = 80 %, Brown et al. Prediction of in vivo drug-drug interactions from in vitro data: impact of incorporating parallel pathways of drug elimination and inhibitor absorption rate constant. Br J Clin Pharmacol. 2005 Nov; 60(5):508-18). L'augmentation d'exposition de l'ibuprofène et de la tolbutamide étant très similaire après co-administration du même inhibiteur chez l'homme, on peut conclure que la contribution du CYP2C9 dans le métabolisme de ces deux molécules est très similaire. Les résultats obtenus avec le kit de la présente invention sont confortés (Tableau 4) et démontrent l'excellente représentativité de ce modèle *in vitro* par rapport à la situation en clinique.

**Tableau 4 : Pourcentage d'implication des CYP450 dans le métabolisme oxydatif de l'ibuprofène à partir du kit de microsomes isolés irréversiblement inhibés selon l'invention, sur enzymes recombinantes humaines (McGinnity et al.) et en situation in vivo chez le sujet sain (Hynninen et al., Lazar et al. et Brown et al.).**

| CYP450 impliqué | % d'implication des CYP450 dans le métabolisme oxydatif de l'Ibuprofene | | | | |
|---|---|---|---|---|---|
| | Kit de microsomes isolés | Données attendues¹ | Données attendues² | Données attendues³ | Données attendues⁴ |
| CYP2C9 | 90 | 91 | 45 | 52 | (80) |

| | | | | | |
|---|---|---|---|---|---|
| ¹ de McGinnity et al (2000) sur modèle d'enzymes humaines recombinantes ² de Hynninen et al (2006) ³ de Lazar JD et al (1990) ⁴ de H.S. Brown et al, (2005) | | | | | |

### • Celecoxib

Le celecoxib a été incubé selon les conditions décrites précédemment avec une concentration en protéines microsomales de 2 mg/ml permettant une mesure optimale de sa clairance intrinsèque. En présence des microsomes témoins (non inhibés et préparés selon l'invention), une clairance intrinsèque de 13.4 à 18.9 ml/min/g protéines a été mesurée. Par rapport aux microsomes témoins, une inhibition de la clairance intrinsèque du celecoxib de 81 % a été mise évidence en présence des microsomes de foie isolés irréversiblement inhibés pour le CYP450 2C9 (Figures 21 et 22). Aucune inhibition significative de la clairance intrinsèque de la celecoxib n'a été observée en présence des microsomes de foie isolés irréversiblement inhibés pour les CYP450 1A2, 2B6, 2D6, 2C8 et 3A4. Par inhibition significative, on entend une clairance intrinsèque inférieure à 25%, pourcentage représentant le seuil de variabilité observée sur des mesures de clairance sur microsomes de foie. En conséquence, le métabolisme oxydatif du celecoxib implique le CYP450 2C9 à hauteur de 81 %.

Une étude chez le volontaire sain montre que l'administration orale répétée de fluconazole à 200 mg, augmente de 134% l'exposition (AUC) en celecoxib co-administré per os à 200 mg (NDA020998 1998-12-31 Pharmacia).

L'augmentation d'exposition de celecoxib et de la tolbutamide citée précédemment (Lazar et al.) étant très similaire après co-administration de la fluconazole chez l'homme, on peut conclure que la contribution du CYP2C9 dans le métabolisme de ces deux principes actifs est très similaire. Brown et al. ont montré pour la tolbutamide une implication du CYP2C9 de 80 %. Les résultats obtenus avec le kit de microsomes isolés irréversiblement inhibés selon l'invention démontrent la bonne représentativité de ce modèle *in vitro* par rapport à la situation en clinique (Tableau 5).

**Tableau 5 : Pourcentage d'implication des CYP450 dans le métabolisme oxydatif du celocoxib à partir du kit de microsomes isolés irréversiblement inhibés selon l'invention et en situation in vivo chez le sujet sain (NDA020998, Lazar et al. et Brown et al.)**

| CYP450 impliqué | % d'implication des CYP450 dans le métabolisme oxydatif du Celocoxib | | | |
|---|---|---|---|---|
| | Kit de microsomes isolés | Données attendues¹ | Données attendues² | Données attendues³ |
| CYP2C9 | 81 | 57 | 52 | (80) |

| | | | | |
|---|---|---|---|---|
| ¹ de NDA020998 1998-12-31 (Pharmacia) ² de Lazar JD et al (1990) ³ de H.S. Brown et al, (2005) | | | | |

### • Pioglitazone

La pioglitazone a été incubée selon les conditions décrites précédemment avec une concentration en protéines microsomales de 0,2 mg/ml permettant une mesure optimale de sa clairance intrinsèque. En présence des microsomes témoins (non inhibés et préparés selon l'invention), une clairance intrinsèque de 43 à 70 ml/min/g protéines a été mesurée. Par rapport aux microsomes témoins, une inhibition de la clairance intrinsèque de la pioglitazone de 69% a été mise évidence en présence des microsomes de foie isolés irréversiblement inhibés pour le CYP450 2C8 (Figures 23 et 24).

Aucune inhibition significative de la clairance intrinsèque de la pioglitazone n'a été observée en présence des microsomes de foie isolés irréversiblement inhibés pour les CYP450 1A2, 2B6, 2D6, 2C9 et 3A4. Par inhibition significative, on entend une clairance intrinsèque inférieure à 25%, pourcentage représentant le seuil de variabilité observée sur des mesures de clairance sur microsomes de foie. En conséquence, le métabolisme oxydatif de la pioglitazone implique le CYP450 2C8 à hauteur de 69%.

Une étude chez le volontaire sain montre que l'administration orale du gemfibrosil à 600 mg, inhibiteur du CYP2C8, augmente de 239% l'exposition (AUC) en pioglitazone co-administrée per os à 3 mg (Deng et al. Effect of gemfibrozil on the pharmacokinetics of pioglitazone. Eur J Clin Pharmacol, 2005, 61, 831-6). Cette augmentation de l'exposition correspond à une implication du CYP2C8 estimée à 71% de la clairance totale de la pioglitazone. Les résultats obtenus avec le kit selon l'invention corroborent donc les données décrites en situation clinique par Deng et al. (Tableau 6).

**Tableau 6 : Pourcentage d'implication des CYP450 dans le métabolisme oxydatif de la pioglitazone obtenu à partir du kit de microsomes isolés irréversiblement inhibés selon l'invention et en situation in vivo (Deng et al.)**

| CYP450 impliqué | % d'implication des CYP450 dans le métabolisme oxydatif du Pioglitazone | |
|---|---|---|
| | Kit de microsomes isolés | Données attendues¹ |
| CYP2C8 | 69 | 71 |

| | | |
|---|---|---|
| ¹ de Deng LJ et al, 2005 sur étude in vivo | | |

### • Bortezomib

Le bortezomib a été incubé selon les conditions décrites précédemment avec une concentration en protéines microsomales de 1,5 mg/ml permettant une mesure optimale de sa clairance intrinsèque. En présence des microsomes témoins (non inhibés et préparés selon l'invention), une clairance intrinsèque de 6,9 à 11 ml/min/g protéines a été mesurée. Par rapport aux microsomes témoins, une inhibition de la clairance intrinsèque du bortezomib de 73% a été mise évidence en présence des microsomes de foie isolés irréversiblement inhibés pour le CYP450 3A4 (Figures 25 et 26).

Aucune inhibition significative de la clairance intrinsèque du bortezomib n'a été observée en présence des microsomes de foie isolés irréversiblement inhibés pour les CYP450 1A2, 2B6, 2D6, 2C8 et 2C9. Par inhibition significative, on entend une clairance intrinsèque inférieure à 25%, pourcentage représentant le seuil de variabilité observée sur des mesures de clairance sur microsomes de foie. En conséquence, le métabolisme oxydatif du bortezomib implique le CYP450 3A4 à hauteur de 73%. Uttamsingh et al. (Relative contributions of the five major human cytochromes p450, 1A2, 2C9, 2C19, 2D6, and 3A4, to the hepatic metabolism of the proteasome inhibitor bortezomib. Drug Metab Dipos 2005, 33 (11):1723-1728) ont montré qu'un anticorps monoclonal anti-CYP 3A4 inhibe 79% du métabolisme du bortézomib (2 µM) par les microsomes de foie humain. Les résultats obtenus avec le kit décrit dans la présente invention corroborent donc les données décrites par Uttamsingh et al. (Tableau 7).

**Tableau 7 : Pourcentage d'implication des CYP450 dans le métabolisme oxydatif du bortezomib obtenu à partir du kit de microsomes isolés irréversiblement inhibés selon l'invention et à partir de microsomes hépatiques humains inhibés par des anticorps monoclonaux spécifiques (Uttamsingh et al.)**

| CYP450 impliqué | % d'implication des CYP450 dans le métabolisme oxydatif du Bortezomib | |
|---|---|---|
| | Kit de microsomes isolés | Données attendues¹ |
| CYP3A4 | 73 | 79 |

| | | |
|---|---|---|
| ¹ de Uttamsingh et al (2005) sur modèle de microsomes hépatiques humains (utilisation des anticorps monoclonaux) | | |

### • Repaglinide

La repaglinide a été incubée selon les conditions décrites précédemment avec une concentration en protéines microsomales de 2 mg/ml permettant une mesure optimale de sa clairance intrinsèque. En présence de microsomes témoins (non inhibés et préparés selon l'invention), une clairance intrinsèque de 38.4 à 48.9 ml/min/g protéines a été mesurée. Par rapport au microsomes témoins, une inhibition de la clairance intrinsèque de la repaglinide de 80% a été mise évidence en présence des microsomes de foie isolés irréversiblement inhibés pour le CYP450 2C8 (Figures 27 et 28). Aucune inhibition significative de la clairance intrinsèque de la repaglinide n'a été observée en présence des microsomes de foie isolés irréversiblement inhibés pour les CYP450 1A2, 2B6, 2D6, 2C9 et 3A4. Par inhibition significative, on entend une clairance intrinsèque inférieure à 25%, pourcentage représentant le seuil de variabilité observée sur des mesures de clairance sur microsomes de foie. En conséquence, le métabolisme oxydatif de la repaglinide implique le CYP450 2C8 à hauteur de 80%.

Une étude chez le volontaire sain montre que l'administration orale du gemfibrosil, (jusqu'à 900 mg), inhibiteur du CYP2C8, augmente de 8.3 fois l'exposition (AUC) en repaglinide co-administrée per os à 0.25 mg (Honkalammi J. et al. Dose-Dependent Interaction between gemfibrozil and repaglinide in humans: strong inhibition of CYP2C8 with subtherapeutic gemfibrozil doses. Drug Metab Dispos, 2011, 39, 1977-1986). Cette augmentation de l'exposition correspond à une implication du CYP2C8 estimée de 88% de la clairance totale de la repaglinide. Les résultats obtenus avec le kit de microsomes isolés irréversiblement inhibés selon l'invention sont corroborés par les données décrites en situation clinique par Honkalammi J. et al. (Tableau 8).

**Tableau 8 : Pourcentage d'implication des CYP450 dans le métabolisme oxydatif de la répaglidine obtenu à partir du kit de microsomes isolés irréversiblement inhibés selon l'invention et en situation in vivo chez le sujet sain (Honkalammi J. et al.)**

| CYP450 impliqué | % d'implication des CYP450 dans le métabolisme oxydatif de la Répaglinide | |
|---|---|---|
| | Kit de microsomes isolés | Données attendues¹ |
| CYP2C8 | 80 | 88 |

| | | |
|---|---|---|
| ¹ de Honkalammi J. et al, 2011. | | |

### • Sertraline

La sertraline a été incubée selon les conditions décrites précédemment avec une concentration en protéines microsomales de 0.2 mg/ml permettant une mesure optimale de sa clairance intrinsèque. En présence de microsomes témoins (non inhibés et préparés selon l'invention), une clairance intrinsèque de 52.5 à 70.5 ml/min/g protéines a été mesurée. Par rapport aux microsomes témoins, une inhibition de la clairance intrinsèque de la sertraline de 58% a été mise évidence en présence des microsomes de foie isolés irréversiblement inhibés pour le CYP450 2B6 (Figures 29 et 30). Il est également observé une inhibition de la clairance intrinsèque de la sertraline de 64% en présence des microsomes de foie isolés irréversiblement inhibés pour le CYP2D6. Sachant que la paroxetine, inhibiteur MBI du CYP2D6, n'est pas spécifique et inhibe également le CYP2B6, on en déduit donc que l'inhibition du CYP2D6 correspond en réalité à celle du CYP2B6.

Aucune inhibition significative de la clairance intrinsèque de la sertraline n'a été observée en présence des microsomes de foie isolés irréversiblement inhibés pour les CYP450 1A2, 2C8, 2C9 et 3A4. Par inhibition significative, on entend une clairance intrinsèque inférieure à 25%, pourcentage représentant le seuil de variabilité observée sur des mesures de clairance sur microsomes de foie. En conséquence, le métabolisme oxydatif de la sertraline implique le CYP450 2B6 à hauteur de 58%.

Après incubation de la sertraline en présence de microsomes de foies humains et d'inhibiteurs spécifiques des CYP450, Obach S et al. (Sertraline is metabolized by multiple cytochrome P450 enzymes, monoamine oxidases, and glucuronyl transferases in human: an in vitro study. Drug Metab Dispos. 2005 Feb;33(2):262-70) ont montré que parmi les CYP450 majoritaires, le CYP2B6 contribue le plus dans le métabolisme de la sertraline avec 15 à 65% d'implication (60 % sur un pool de foies humains). Les résultats obtenus avec le kit décrit selon la présente invention sont corroborés par les résultats décrits par Obach S et al. (Tableau 9).

**Tableau 9 : Pourcentage d'implication des CYP450 dans le métabolisme oxydatif de la sertraline obtenu à partir du kit de microsomes isolés irréversiblement inhibés et sur microsomes de foies humains en présence ou non d'inhibiteurs spécifiques des CYP450 (Obach S et al.)**

| | % d'implication des CYP450 dans le métabolisme oxydatif de la Sertraline | |
|---|---|---|
| CYP450 impliqué | Kit de microsomes isolés | Données attendues¹ |
| CYP2B6 | 58 | 15 à 65 - 60 |

| | | |
|---|---|---|
| ¹ de Obach S et al (2004) sur des microsomes de foies humains +/- inhibiteur specifique de CYP450 | | |

Les résultats obtenus montrent que la contribution des enzymes impliquées dans le métabolisme des principes actifs sélectionnés, mesurée *in vitro* avec le kit de phénotypage sont très similaires voire identiques avec ceux estimés ou mesurés à partir de données *in vivo* et/ou à partir de données issues d'autres modèles *in vitro.* La validation du kit de microsomes cryoconservés, isolés et irréversiblement inhibés dans le cadre d'un phénotypage enzymatique des voies métaboliques d'un xénobiotique par rapport aux données cliniques démontre la représentativité de ce modèle *in vitro* par rapport à la situation *in vivo* chez l'homme.

De plus, l'acquisition d'une mesure directe de la contribution enzymatique dans le métabolisme d'un principe actif, permet non seulement un gain de temps et une facilité d'interprétation mais évite également des erreurs inhérentes à la multiplication des manipulations pour la mise en oeuvre des autres modèles *in vitro.*

## Revendications

1. Méthode de préparation de microsomes isolés et conservés, qui comprennent un cytochrome P450 (CYP450) irréversiblement inhibé, pour leur utilisation extemporanée **caractérisé en ce qu'**elle comprend les étapes a) à c) dans l'ordre suivant :
a) l'inhibition irréversible d'un cytochrome P450 ;
b) la concentration des protéines microsomales ; et
c) la conservation des microsomes isolés.

2. Méthode selon la revendication 1, **caractérisée en ce qu'**elle comprend une à plusieurs étapes de lavage.

3. Méthode selon la revendication 2, **caractérisée en ce que** la ou les étapes de lavage sont placées avant et/ou après l'étape de concentration des protéines microsomales.

4. Méthode selon l'une quelconque des revendications 1 à 3, **caractérisée en ce que** la concentration des microsomes est obtenue par filtration/centrifugation ou ultracentrifugations.

5. Méthode selon l'une quelconque des revendications 1 à 4, **caractérisée en ce que** les microsomes sont concentrés à une concentration de 10mg/ml à 30mg/ml.

6. Méthode selon l'une quelconque des revendications 1 à 5, **caractérisée en ce que** l'étape de conservation est la congélation.

7. Méthode selon l'une quelconque des revendications 1 à 6, **caractérisée en ce que** les microsomes sont des microsomes de foie humains.

8. Méthode selon l'une quelconque des revendications 1 à 7, **caractérisée en ce que** le cytochrome P450 irréversiblement inhibé est sélectionné dans les familles CYP1, CYP2 et CYP3.

9. Méthode selon la revendication 8, **caractérisée en ce que** le cytochrome P450 est sélectionné parmi la liste des cytochromes suivants : CYP1A2, CYP2A6, CYP2B6, CYP2C8, CYP2C9, CYP2C19, CYP2D6, CYP2E1, CYP3A4.

## Patentansprüche

1. Verfahren zur Herstellung von isolierten und konservierten Mikrosomen, die ein irreversibel inhibiertes Cytochrom P450 (CYP450) umfassen, für die Zubereitung unmittelbar vor ihrer Verwendung, **dadurch gekennzeichnet, dass** es die Stufen a) bis c) in der folgenden Reihenfolge umfasst:
a) die irreversible Inhibierung eines Cytochroms P450;
b) die Konzentration von mikrosomalen Proteinen; und
c) die Konservierung der isolierten Mikrosomen.

2. Verfahren nach Anspruch 1, **dadurch gekennzeichnet, dass** es ein bis mehrere Waschstufen umfasst.

3. Verfahren nach Anspruch 2, **dadurch gekennzeichnet, dass** die Waschstufe(n) vor und/oder nach der Stufe der Konzentration der mikrosomalen Proteine durchgeführt wird (werden).

4. Verfahren nach einem der Ansprüche 1 bis 3, **dadurch gekennzeichnet, dass** die Konzentration der Mikrosomen durch Filtration/Zentrifugation oder Ultrazentrifugationen erfolgt.

5. Verfahren nach einem der Ansprüche 1 bis 4, **dadurch gekennzeichnet, dass** die Mikrosomen auf eine Konzentration von 10 mg/ml bis 30 mg/ml konzentriert werden.

6. Verfahren nach einem der Ansprüche 1 bis 5, **dadurch gekennzeichnet, dass** die Stufe der Konservierung das Einfrieren ist.

7. Verfahren nach einem der Ansprüche 1 bis 6, **dadurch gekennzeichnet, dass** die Mikrosomen menschliche Lebermikrosomen sind.

8. Verfahren nach einem der Ansprüche 1 bis 7, **dadurch gekennzeichnet, dass** das irreversibel inhibierte Cytochrom P450 aus den Familien CYP1, CYP2 und CYP3 ausgewählt wird.

9. Verfahren nach Anspruch 8, **dadurch gekennzeichnet, dass** das Cytochrom P450 ausgewählt wird aus der folgenden Liste von Cytochromen: CYP1A2, CYP2A6, CYP2B6, CYP2C8, CYP2C9, CYP2C19, CYP2D6, CYP2E1, CYP3A4.

## Claims

1. Method for preparing isolated and preserved microsomes which comprise an irreversibly inhibited cytochrome P450 (CYP450), for extemporaneous use, **characterised in that** it comprises steps a) to c) in the following order:
a) irreversible inhibition of a cytochrome P450;
b) concentration of the microsomal proteins; and
c) preservation of the isolated microsomes.

2. Method according to claim 1, **characterised in that** it comprises one or more washing steps.

3. Method according to claim 2, **characterised in that** the washing step(s) is/are placed before and/or after the step of concentration of the microsomal proteins.

4. Method according to any one of claims 1 to 3, **characterised in that** concentration of the microsomes is obtained by filtration/centrifugation or ultracentrifugations.

5. Method according to any one of claims 1 to 4, **characterised in that** the microsomes are concentrated to a concentration of from 10 mg/ml to 30 mg/ml.

6. Method according to any one of claims 1 to 5, **characterised in that** the step of preservation is freezing.

7. Method according to any one of claims 1 to 6, **characterised in that** the microsomes are human liver microsomes.

8. Method according to any one of claims 1 to 7, **characterised in that** the irreversibly inhibited cytochrome P450 is selected from the families CYP1, CYP2 and CYP3.

9. Method according to claim 8, **characterised in that** the cytochrome P450 is selected from the list of the following cytochromes: CYP1A2, CYP2A6, CYP2B6, CYP2C8, CYP2C9, CYP2C19, CYP2D6, CYP2E1, CYP3A4.
